(19) Europäisches Patentamt
European Patent Office
Office européen des brevets

(11) Veröffentlichungsnummer: **0 166 692**
**B1**

(12) # EUROPÄISCHE PATENTSCHRIFT

(45) Veröffentlichungstag der Patentschrift: **11.07.90**

(51) Int. Cl.$^5$: **C 07 D 221/22,** A 61 K 31/435

(21) Anmeldenummer: **85810275.9**

(22) Anmeldetag: **14.06.85**

(54) **Substituierte Azabicycloheptane, ihre Verwendung, pharmazeutische Präparate, welche diese Verbindungen enthalten, und Verfahren zur Herstellung dieser Verbindungen.**

(30) Priorität: **20.06.84 CH 2987/84**

(43) Veröffentlichungstag der Anmeldung:
**02.01.86 Patentblatt 86/01**

(45) Bekanntmachung des Hinweises auf die Patenterteilung:
**11.07.90 Patentblatt 90/28**

(84) Benannte Vertragsstaaten:
**AT BE CH DE FR GB IT LI LU NL SE**

(56) Entgegenhaltungen:
**BE-A- 548 074**
**DE-B-1 088 957**

**JOURNAL OF THE AMERICAN CHEMICAL SOCIETY, Band 105, 1983, Seiten 6712-6714, American Chemical Society, Columbus, Ohio, US; A. ALDER et al.: "Intramolecular thermal cyclization reactions of diacryloylamines"**

(73) Patentinhaber: **CIBA-GEIGY AG**
**Klybeckstrasse 141**
**CH-4002 Basel (CH)**

(72) Erfinder: **Alder, Alex, Dr.**
**Markgräflerstrasse 65/4**
**CH-4057 Basel (CH)**
Erfinder: **Stanek, Jaroslav, Dr.**
**Florastrasse 6**
**CH-4127 Birsfelden (CH)**
Erfinder: **Bellus, Daniel, Dr.**
**Unterm Schellenberg 81**
**CH-4125 Riehen (CH)**

**EP 0 166 692 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Gegenstand der vorliegenden Erfindung sind neue Aminophenyl-substituierte Azabicycloalkane mit wertvollen pharmakologischen Eigenschaften und Salze von diesen Verbindungen, die Verwendung von diesen Stoffen bzw. von pharmazeutischen Präparaten, welche diese Stoffe enthalten, pharmazeutischen Präparate und Verfahren zur Herstellung dieser neuen Stoffe, sowie Zwischenprodukte und Verfahren zur Herstellung dieser Zwischenprodukte.

In der Deutschen Auslegeschrift 1088957 sind α-(p-Aminophenyl)-α-alkyl-glutarimide mit antikonvulsiver Wirkung beschrieben. Die Erfindungsgemässen Verbindungen unterscheiden sich von den bekannten Glutarimiden dadurch, dass ein bicyclisches Imid ein angular-substituiertes monocyclisches Imid im Molekul ersetzt. In der J. Am. Chem. Soc. *105,* 6712 (1983) wird die Herstellung von substituierten 3-Azabicyclo[3.3.1]heptan-2,4-dionen, insbesondere von 3-(3,5-Dichlorphenyl)-1-methyl-5-phenyl-3-aza-bicyclo[3.3.1]heptan-2,4-dion durch thermische Cyclisierung der entsprechenden Diacrylimide beschrieben. Diese bekannten ohne Wirkungsangaben charakterisierten Verbindungen entbehren der essentiellen Aminogruppe im Phenylrest.

Die vorliegende Erfindung betrifft substituierte 1-Phenyl-3-azabicyclo[3.3.1]heptan-2,4-dione der Formel

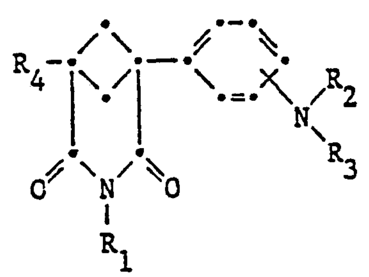

(I),

worin $R_1$ Wasserstoff, Alkyl mit 1—12 C-Atomen, Alkenyl mit 2—12 C-Atomen, Niederalkinyl, Cycloalkyl oder Cycloalkenyl mit 3—10 C-Atomen, Cycloalkylniederalkyl mit 4—10 C-atomen, Cycloalkylniederalkenyl mit 5—10 C-Atomen, Cycloalkenylniederalkyl mit 4—10 C-Atomen oder unsubstituiertes oder durch Niederalkyl, Hydroxy, Niederalkoxy, Acyloxy, Amino, Niederalkylamino, Diniederalkylamino, Acylamino oder Halogen substituiertes Aryl mit 6—12 C-Atomen oder Arylniederalkyl mit 7—15 C-Atomen, $R_2$ Wasserstoff, Niederalkyl, Sulfo, Niederalkanoyl oder Niederalkansulfonyl, $R_3$ Wasserstoff oder Niederalkyl und $R_4$ Wasserstoff, Niederalkyl, Phenyl oder durch —N($R_2$)($R_3$) substituiertes Phenyl bedeuten, sowie Salze davon, wobei die mit "nieder" bezeichneten Reste bis einschliesslich 7 Kohlenstoffatome enthalten.

In der Beschreibung der vorliegenden Erfindung bedeutet der bei der Definition von Gruppen oder Resten, z.B. Niederalkyl, Niederalkoxy, Niederalkanoyl etc., verwendete Ausdruck "Nieder", dass die so definierten Gruppen oder Reste, falls nicht ausdrücklich anders definiert, bis einschliesslich 7 und bevorzugt bis einschliesslich 4 C-Atome enthalten.

Die in der Beschreibung verwendeten allgemeinen Ausdrücke und Bezeichnungen haben vorzugsweise die folgenden Bedeutungen:

Die Gruppe —N($R_2$)($R_3$) kann sich in 2-, 3- oder 4-Stellung des Phenylrests befinden.

Alkyl $R_1$ hat 1—12 C-Atome und ist z.B. Niederalkyl mit 1—7 C-Atomen, z.B. Methyl, Aethyl, n-Propyl, Isopropyl, n-Butyl, Isobutyl, tert-Butyl, n-Pentyl, Neopentyl, n-Hexyl oder n-Heptyl, sowie n-Octyl, n-Nonyl, n-Decyl, n-Undecyl oder n-Dodecyl.

Alkenyl $R_1$ hat 2—12 C-Atome und ist z.B. Niederalkenyl mit 3—7 C-Atomen, z.B. Allyl oder 2- oder 3-Butenyl, sowie 2-Octenyl, 2-Nonenyl, 2-Decenyl, 2-Undecenyl oder 2-Dodecenyl, wobei die Doppelbindung sich auch in einer anderen als der 2-Stellung befinden kann.

Niederalkinyl $R_1$ hat 2—7, insbesondere 3—4, C-Atome und ist z.B. 2-Propinyl oder 2-Butinyl.

Cycloalkyl $R_1$ hat 3—10, insbesondere 3—6, C-Atome und ist z.b. Cyclopropyl, Cyclobutyl, Cyclopentyl oder Cyclohexyl.

Cycloalkenyl $R_1$ hat 3—10, insbesondere 3—6, C-Atome und ist z.B. 2-Cyclohexenyl oder 2,5-Cyclohexadienyl.

Cycloalkylniederalkyl $R_1$ hat 4—10, insbesondere 4—7, C-Atome und ist z.B. Cyclopropylmethyl, Cyclobutylmethyl, Cyclopentylmethyl oder Cyclohexylmethyl, sowie 2-Cyclopropyläthyl, 2-Cyclobutyläthyl, 2-Cyclopentyläthyl oder 2-Cyclohexyläthyl.

Cycloalkylniederalkenyl $R_1$ hat 5—10, insbesondere 4—9, C-Atome und ist z.B. Cyclohexylvinyl oder Cyclohexylallyl.

Cycloalkenylniederalkyl $R_1$ hat 4—10, insbesondere 4—8 C-Atome und ist z.B. 1-Cyclohexenylmethyl oder 1,4-Cyclohexadienylmethyl, sowie 2-(1-Cyclohexenyl)-äthyl oder 2-(1,4-Cyclohexadienyl)-äthyl.

Unsubstituiertes oder substituiertes Aryl $R_1$ hat 6—12 C-Atome und ist z.B. Phenyl, 1- oder 2-Naphthyl, gegebenenfalls substituiert durch Niederalkyl, Hydroxy, Niederalkoxy, Acyloxy, Amino, Niederalkylamino, Diniederalkylamino, Acylamino oder Halo, wobei die Substituenten in 2-, 3- oder 4-Stellung des

Phenylrings stehen und auch mehrfach vorkommen können, z.B. 4-Methylphenyl, 4-Methyl-1-naphthyl, 4-Hydroxyphenyl, 3- oder 4-Methoxyphenyl, 3,4-Dimethoxyphenyl, 4-Acetoxyphenyl, 3- oder 4-Dimethylaminophenyl, 4-Acetaminophenyl, 3- oder 4-Chlorphenyl oder 4-Bromphenyl.

Unsubstituiertes oder substituiertes Arylniederalkyl $R_1$ hat 7—15 C-Atome und ist z.B. Benzyl, 2-Phenyläthyl oder 1- oder 2-Naphthylmethyl, wobei der Arylrest durch die gleichen Gruppen substituiert sein kann wie in Aryl $R_1$, z.B. 4-Methylbenzyl, 4-Methoxybenzyl, 3,4-Dimethoxybenzyl, 2-(4-Methoxyphenyl)-äthyl oder 4-Dimethylaminobenzyl.

Niederalkyl $R_2$ oder $R_3$ hat die unter $R_1$ genannten Bedeutungen und ist vorzugsweise Methyl oder Aethyl.

Acyl $R_2$ ist als Niederalkanoyl, z.B. Formyl oder Acetyl, oder Niederalkansulfonyl, z.B. Methan- oder Aethansulfonyl.

Niederalkyl $R_4$ ist z.B. Methyl, Aethyl, n-Propyl, Isopropyl, n-Butyl, Isobutyl, tert-Butyl, n-Pentyl, Neopentyl, n-Hexyl oder n-Heptyl.

In einem durch —$N(R_2)(R_3)$ substituierten Phenylrest $R_4$ haben $R_2$ und $R_3$ die oben angegebenen Bedeutungen, wobei der Phenylring in 2-, 3- oder 4-Stellung substituiert sein kann.

Salze von erfindungsgemässen Verbindungen der Formel I mit einer salzbildenden Gruppe sind in erster Linie pharmazeutisch verwendbare, nicht-toxische Salze.

Solche Salze werden beispielsweise von der Aminogruppe am Phenylring durch Addition einer anorganischen Säure, z.B. Salzsäure, Schwefelsäure, Niederalkansulfonsäure oder Phosphorsäure gebildet und sind beispielsweise Hydrochloride, Hydrogensulfate, Methansulfonate, Hydrogenphosphate oder Dihydrogenphosphate.

Weitere Säureadditions salze werden beispielsweise von Carbonsäuren gebildet und sind beispielsweise Formiate, Acetate, Trifluoracetate, Benzoate, Citrate, Tartrate oder Salicylate.

Die Verbindungen der Formel I können auch als Hydrate vorliegen.

Die vorliegende Erfindung betrifft hauptsächlich Verbindungen der Formel I, worin $R_1$ Wasserstoff, Alkyl bis einschliesslich 12 C-Atome, z.B. Niederalkyl, z.B. Methyl, Aethyl, n-Propyl, Isopropyl, n-Butyl, Isobutyl, tert-Butyl, n-Pentyl, Neopentyl, n-Hexyl oder n-Heptyl, sowie n-Octyl, n-Nonyl, n-Decyl, n-Undecyl oder n-Dodecyl, Niederalkenyl, z.B. Allyl oder 2-Butenyl, Niederalkinyl, z.B. 2-Propinyl oder 2-Butinyl, Cycloalkyl, z.B. Cyclopropyl, Cyclopentyl oder Cyclohexyl, Cycloalkylniederalkyl, z.B. Cyclopentylmethyl oder Cyclohexylmethyl, oder unsubstituiertes oder substituiertes Arylniederalkyl, z.B. Benzyl oder 4-Methoxybenzyl, $R_2$ Wasserstoff, Niederalkyl, z.B. Methyl, Niederalkanonyl, z.B. Acetyl, oder Niederalkansulfonyl, z.B. Methansulfonyl, $R_3$ Wasserstoff oder Niederalkyl, z.B. Methyl, und $R_4$ Wasserstoff, Niederalkyl, z.b. Methyl, Aethyl, n-Propyl, Isopropyl, n-Butyl, Isobutyl oder tert-Butyl, Phenyl oder durch —$N(R_2)(R_3)$ substituiertes Phenyl bedeuten, sowie Salze davon, insbesondere pharmazeutisch verwendbare Salze davon.

Die vorliegende Erfindung betrifft in erster Linie Verbindungen der Formel I, worin $R_1$ Wasserstoff, Niederalkyl, z.B. Methyl, Aethyl, n-Propyl, Isopropyl, n-Butyl, Isobutyl, tert-Butyl, n-Pentyl, Neopentyl, n-Hexyl oder n-Heptyl, Niederalkenyl, z.B. Allyl, Niederalkinyl, z.B. 2-Propinyl, Cycloalkyl, z.b. Cyclohexyl, Cycloalkylniederalkyl, z.B. Cyclohexylmethyl, $R_2$ und $R_3$ Wasserstoff und $R_4$ Wasserstoff, Niederalkyl, z.B. Methyl, Aethyl, n-Propyl, Isopropyl, n-Butyl, Isobutyl oder tert-Butyl, Phenyl oder durch —$N(R_2)(R_3)$ substituiertes Phenyl bedeuten, sowie pharmazeutisch verwendbare Salze davon.

Die vorliegende Erfindung betrifft vor allem Verbindungen der Formel I, worin die Gruppe —$N(R_2)(R_3)$ sich in 4-Stellung des Phenylrests befindet, $R_1$ Wasserstoff, Niederalkyl, z.B. Methyl, Aethyl, n-Propyl, Isopropyl, n-Butyl, Isobutyl oder tert-Butyl, Niederalkenyl, z.B. Allyl, Niederalkinyl, z.B. 2-Propinyl, Cycloalkylniederalkyl, z.B. Cyclohexylmethyl, $R_2$ und $R_3$ Wasserstoff und $R_4$ Wasserstoff oder Niederalkyl, z.B. Methyl, Aethyl, n-Propyl, Isopropyl, n-Butyl, Isobutyl oder tert-Butyl, bedeuten, sowie pharmazeutisch verwendbare Salze davon.

Ganz besonders betrifft die Erfindung Verbindungen der Formel I, worin die Gruppe —$N(R_2)(R_3)$ sich in 4-Stellung des Phenylrests befindet, $R_1$ Wasserstoff, Alkyl bis einschliesslich 12 C-Atome, z.B. Niederalkyl, wie Methyl, Aethyl, n-Propyl, Isopropyl, n-Butyl, Isobutyl, n-Pentyl, neo-Pentyl, n-Hexyl oder n-Heptyl, sowie n-Octyl, n-Nonyl, n-Decyl, n-Undecyl oder n-Dodecyl, Niederalkenyl, z.B. Allyl, Niederalkinyl, z.B. 2-Propinyl, Cycloalkyl, z.B. Cyclopentyl oder Cyclohexyl, Cycloalkylmethyl, z.B. Cyclopentylmethyl oder Cyclohexylmethyl, Benzyl oder durch Niederalkyl, Hydroxy, Niederalkoxy oder Niederalkanoyloxy substituiertes Benzyl, z.B. 4-Methoxybenzyl, und $R_2$, $R_3$ und $R_4$ Wasserstoff bedeuten, sowie pharmazeutisch verwendbare Salze davon.

Die vorliegende Erfindung hat vor allem die in den Beispielen genannten Verbindungen zum Gegenstand.

Die Erfindung betrifft ebenfalls pharmazeutische Präparate enthaltend Verbindungen der Formel I, worin $R_1$, $R_2$, $R_3$ und $R_4$ die oben angegebenen Bedeutungen haben, und die Anwendung dieser Verbindungen in einem Verfahren zur Behandlung des menschlichen und tierischen Körpers.

Die neuen Verbindungen der Formel I und ihre pharmazeutisch verwendbaren Salze besitzen beispielsweise als Aromatasehemmer wertvolle pharmakologische Eigenschaften. Die Eignung von Verbindungen der Formel I als Aromatasehemmer kann im "Aromatase Assay" nach Graves P. E. und Salhanick H. A., Endocrinology Bd. 105, S. 52 (1979) bei Verwendung von humanen, placentalen Mikrosomen in vitro nachgewiesen werden. In dieser Versuchsanordnung wird die Bildung von Wasser mit Tritiumisotopen und 17β-Oestradiol aus [1β,2β-$^3$H]-Testosteron als Folge des Einwirkens einer Verbindung

der Formel I auf die Bildung der hydrierten Form des Aromatasecoenzyms Nicotinsäureamid-adenin-dinucleotidphosphat (NADPH) gemessen. Durch Zugabe einer erfindungsgemässen Verbindung der Formel I, beispielsweise von 1-(4-Aminophenyl)-3-n-propyl-3-azabicyclo[3.1.1]heptan-2,4-dion, wird die Enzymaktivität (NADPH-Gehalt) erheblich vermindert, was einen deutlich geringeren Gehalt an Wasser mit radioaktiven Tritiumisotopen als bei Messungen ohne Zugabe einer erfindungsgemässen Verbindung der Formel I zur Folge hat. Vergleichsmessungen zeigen ausserdem, dass die Verringerung der Enzymaktivität als Folge der Zugabe einer erfindunggemässen Verbindung der Formel I, z.B. von 1-(4-Aminophenyl)-3-n-propyl-3-azabicyclo[3.1.1]heptan-2,4-dion, bei gleichen Konzentrationen erheblich stärker ist als bei Zugabe von anderen bekannten Aromatasehemmern, z.B. Aminoglutethimid.

Aufgrund ihrer Wirkung als Aromatasehemmer können die Verbindungen der Formel I, worin $R_1$, $R_2$, $R_3$ und $R_4$ die oben angegebenen Bedeutungen haben oder Salze davon als Heilmittel, beispielsweise in Form von pharmazeutischen Präparaten, bei der Behandlung von hormonabhängigen Krankheiten, z.B. hormonabhängigen Tumoren, wie Tumoren, die von einer Ueberproduktion von Oestrogen abhängig sind, insbesondere Mammakarzinomen, und hormonalen Anomalien, z.B. Gynäkomastie oder Prostatahyperplasie, bei Warmblütern (Menschen und Tiere) durch enterale, z.B. orale, oder parenterale Applikation von therapeutisch wirksamen Dosen angewendet werden.

Die Anwendung dieser Verbindungen als Heilmittel, insbesondere mit carcinostatischer Wirkung, in einem der genannten Verfahren zur Behandlung des menschlichen oder tierischen Körpers ist ebenfalls Gegenstand der vorliegenden Erfindung.

Die zur Anwendung gelangenden täglichen Dosen von solchen Verbindungen betragen für Säugetiere in Abhängigkeit von der Spezies, sowie für Personen abhängig vom Alter, dem individuellen Zustand und der Applikationsweise zwischen etwa 1 mg und etwa 100 mg, insbesondere zwischen 5 mg und etwa 50 mg, pro kg Körpergewicht. Die Dosen sind innerhalb dieses Bereiches bei parenteraler Applikation, z.B. intramuskulärer oder subcutaner Injektion oder intravenöser Infusion, im allgemeinen niedriger als bei enteraler, d.h. oraler oder rektaler Applikation.

Die Verbindungen der Formel I und pharmazeutisch annehmbare Salze von solchen mit salzbildenden Eigenschaften werden oral oder rektal vorzugsweise in Doseneinheitsformen, wie Tabletten, Dragées oder Kapseln bzw. Suppositorien, und parenteral insbesondere als injizierbare Lösungen, Emulsionen oder Suspensionen oder als Infusionslösungen verabreicht, wobei als Lösungen in erster Linie solche von Salzen in Betracht kommen.

Ebenfalls Gegenstand der Erfindung sind pharmazeutische Präparate zur enteralen, z.B. oralen oder rektalen, oder zur parenteralen Verabreichung, welche eine therapeutisch wirksame Menge einer Verbindung der Formel I oder eines pharmazeutisch annehmbaren Salzes einer solchen Verbindung mit einer salzbildenden Gruppe, gegebenenfalls zusammen mit einem pharmazeutisch verwendbaren Trägerstoff oder Trägerstoffgemisch enthalten, wobei als Trägerstoffe feste oder flüssige anorganische oder organische Stoffe verwendet werden. Entsprechende Dosiseinheitsformen, insbesondere zur peroralen Verabreichung, z.B. Dragées, Tabletten oder Kapseln, enthalten zorzugsweise etwa 50 mg bis etwa 500 mg, insbesondere etwa 100 mg bis etw 400 mg einer Verbindung der Formel I oder eines pharmazeutisch annehmbaren Salzes einer zur Salzbilding befähigten entsprechenden Verbindung zusammen mit pharmazeutisch verwendbaren Trägerstoffen.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister unter Verwendung z.B. von Mais-, Weizen-, Reis- oder Kartoffelstärke, Gelatine, Traganth, Methylcellulose und/oder, wenn erwünscht, Sprengmittel, wie die oben genannten Stärken, ferner Carboxymethylstärke, quervernetztes Polyvinylpyrrolidon, Agar, Alginsäure oder ein Salz davon, wie Natriumalginat. Hilfsmittel sind in erster Linie Fliessregulier- und Schmiermittel, z.B. Kieselsäure, Talk, Stearinsäure oder Salze davon, wie Magnesium- oder Calciumstearat, und/oder Polyäthylenglykol. Dragée-Kerne können mit geeigneten, gegebenenfalls Magensaft-resistenten Ueberzügen versehen werden, wobei man u.a. konzentrierte Zuckerlösungen, welche gegebenenfalls arabischen Gummi, Talk, Polyvinylpyrrolidon, Polyäthylenglykol und/oder Titandioxid enthalten, oder Lacklösungen in geeigneten organischen Lösungsmitteln oder Lösungsmittelgemischen, oder, zur Herstellung von Magensaft-resistenten Ueberzügen, Lösungen von geeigneten Cellulosepräparaten, wie Acetylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Weitere oral anwendbare pharmazeutische Präparate sind Steckkapseln aus Gelatine, sowie weiche, geschlossene Kapseln aus Gelatine und einem Weichmacher, wie Glycerin oder Sorbit. Die Steckkapseln können den Wirkstoff in Form eines Granulats, z.B. im Gemisch mit Füllstoffen, wie Lactose, Bindemitteln, wie Stärken und/oder Gleitmitteln, wie Talk oder Magnesiumstearat, und gegebenenfalls von Stabilisatoren, enthalten. In weichen Kapseln ist der Wirkstoff vorzugsweise in geeigneten Flüssigkeiten, wie fetten Oelen, Paraffinöl oder flüssigen Polyäthylenglykolen, gelöst oder suspendiert, wobei ebenfalls Stabilisatoren zugefügt sein können.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetische Triglyceride, Paraffinkohlenwasserstoffe, Polyäthylenglykole oder höhere Alkanole. Ferner können auch Gelatine-Rektalkapseln verwendet werden, die eine Kombination des Wirkstoffs mit einer Grundmasse enthalten; als Grundmassenstoffe kommen z.B. flüssige Triglyceride, Polyäthylenglykole oder Paraffinkohlenwasserstoffe in Frage.

Zur parenteralen Verabreichung eignen sich in erster Linie wässrige Lösungen eines Wirkstoffes in wasserlöslicher Form, z.B. eines wasserlöslichen Salzes, ferner Suspensionen des Wirkstoffes, wie entsprechende ölige Injektionssuspensionen, wobei man geeignete lipophile Lösungsmittel oder Vehikel, wie fette Oele, z.B. Sesamöl, oder synthetische Fettsäureester, z.B. Aethyloleat, oder Triglyceride verwendet, oder wässrige Injektionssuspensionen, welche viskositätserhöhende Stoffe, z.B. Natriumcarboxymethylcellulose, Sorbit und/oder Dextran und gegebenenfalls Stabilisatoren enthalten.

Die pharmazeutischen Präparate der vorliegenden Erfindung können in an sich bekannter Weise, z.B. mittels konventioneller Misch-, Granulier-, Dragier-, Lösungs- oder Lyophilisierungsverfahren hergestellt werden. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig, nach Zugabe von geeigneten Hilfsstoffen, zu Tabletten oder Dragée-Kernen verarbeitet.

Ebenfalls Gegenstand der vorliegenden Erfindung sind Verfahren zur Herstellung von Verbindungen der Formel I und ihrer Salze. Diese können nach an sich bekannten Verfahren hergestellt werden, z.B. indem man

a) in einer Verbindung der Formel

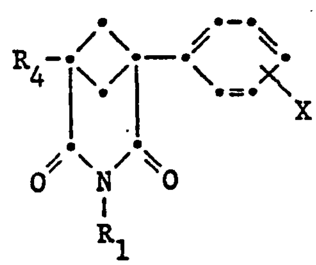

(II),

worin $R_1$ und $R_4$ die unter Formel I genannte Bedeutung haben und X eine Stickstoff enthaltende, reduzierbare Gruppe, Halogen, Carbamoyl, Azidocarbonyl oder eine geschützte Aminogruppe ist, oder in einem Salz davon, X in die $—N(R_2)(R_3)$-Gruppe überführt, oder

b) eine Verbindung der Formel

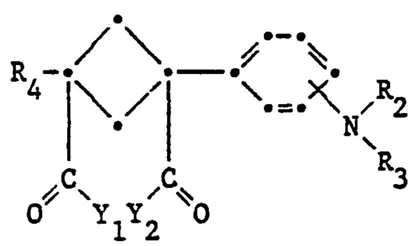

(III),

worin $R_2$, $R_3$ und $R_4$ die unter Formel I genannten Bedeutungen haben und $Y_1$ und $Y_2$ unabhängig voneinander Hydroxy, Halogen, Niederalkoxy, Silyloxy oder Sulfonyloxy oder zusammen Sauerstoff darstellen, oder ein Salz davon durch Umsetzung mit einer die Gruppe $>N—R_1$ liefernden Verbindung zyklisiert, oder

c) eine Verbindung der Formel

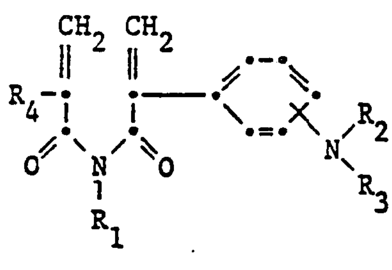

(IV),

worin $R_1$, $R_2$, $R_3$ und $R_4$ die unter Formel I genannte Bedeutung haben, oder ein Salz davon thermisch in Abwesenheit bzw. Anwesenheit eines inerten Lösungsmittels oder auch photochemisch zusammen mit

einem Triplett-Sensibilisatoren in einem photochemisch inerten Lösungsmittel zyklisiert, oder

d) eine Verbindung der Formel

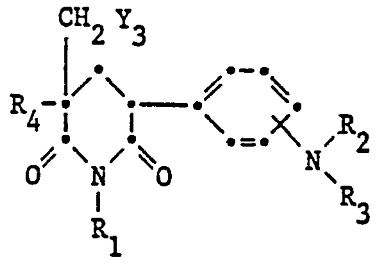

oder

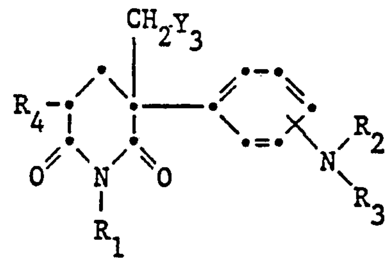

(V)

(VI)

worin $R_1$, $R_2$, $R_3$ und $R_4$ die unter Formel I genannte Bedeutung haben und $Y_3$ Halogen oder Sulfonyloxy bedeutet, oder ein Salz davon in Gegenwert einer nicht-nukleophilen Base zyklisiert,

und gewünschtenfalls ein erhältliches Salz in die freie Verbindung oder in ein anderes Salz überführt, und/oder eine erhältliche freie Verbindung in ein Salz überführt und/oder ein erhältliches Gemisch von Isomeren in die einzelnen Isomere auftrennt.

Verfahren a)

In einer Verbindung der Formel II ist X als eine Stickstoff enthaltende reduzierbare Gruppe, beispielsweise die Nitro-, Nitroso-, Hydroxyamino- oder Azidogruppe, oder eine austauschbare Gruppe, z.B. Halogen, z.B. Chlor, Brom oder Jod, oder eine derivatisierte Carboxylgruppe, z.B. die Carbamoyl- oder Azidocarbonylgruppe, oder eine geschützte Aminogruppe, aus welcher man die Schutzgruppe abspaltet und durch Wasserstoff ersetzt.

Eine Stickstoff enthaltende, reduzierbare Gruppe, z.B. die Nitro-, Nitroso-, Hydroxyamino-, oder Azidogruppe, wird durch ein übliches Reduktionsmittel, welches man gegebenenfalls in Gegenwart eines geeigneten Katalysators und/oder Trägermaterials einsetzt, in die Aminogruppe überführt.

Als übliches Reduktionsmittel kommt in erster Linie in Betracht: Katalytisch aktivierter Wasserstoff, wobei als Hydrierkatalysator beispielsweise ein Edelmetall-, z.B. Palladium-, Platin-, Rhodium- oder Nickelkatalysator, oder eine Edelmetallverbindung, z.B. Platindioxid, verwendet wird und welchen man gegebenenfalls mit einem geeigneten Trägermaterial wie Kohle, Bariumsulfat oder -carbonat oder Calciumcarbonat einsetzt, ein reduzierend wirkendes Zinn(II)- oder Eisen(II)-Salz, die beispielsweise als Chloride und letzteres auch als Sulfat eingesetzt werden, ein reduzierend wirkendes Dithionit- oder Sulfit-Salz, z.B. Natriumdithionit, Natriumsulfit oder Natriumhydrogensulfit, ein unedles, gegebenenfalls aktiviertes Metall, z.B. aktiviertes Eisen, Zinn, Zink oder Aluminium, welches gegebenenfalls in Gegenwart des entsprechenden Metallsalzes oder eines neutralen Salzes, z.B. Calcium-, Magnesium-, Kalium- oder Natriumchlorid aktiviert wird, ferner ein Sulfid, z.B. Schwefelwasserstoff, ein Di- oder Polysulfid, z.B. Natriumdisulfid oder Natriumpolysulfid, ein Alkalimetall- oder Alkalimetallhydrogensulfid, z.B. Natriumsulfid oder Natriumhydrogensulfid, Ammoniumsulfid oder Ammoniumpolysulfid, ein Wasserstoffabgebendes Reduktionsmittel, z.B. unsubstituiertes oder substituiertes Hydrazin, beispielsweise Hydrazin oder Phenylhydrazin, welches gegebenenfalls in Form eines Säureadditionssalzes, z.B. als Hydrochlorid, zugesetzt wird, oder molekularer Wasserstoff, welcher durch elektrolytische Reduktion an der Kathode entladen wird.

Die Reduktion mit katalytisch aktiviertem Wasserstoff erfolgt bei Normaldruck oder erhöhtem Druck, z.B. bis ca. 5 bar. Die Reduktion mit den genannten Reduktionsmitteln erfolgt in saurem, z.B. essigsaurem, oder neutralem Medium. Die Reduktion mit Eisen (II)-Salzen erfolgt unter basischen Bedingungen, wobei das reduzierend wirkende Eisen (II)-hydroxid ausfällt. Ebenfalls unter basischen Bedingungen finden die Reduktionen mit Dithionitsalzen und Sulfiden statt.

Die Reduktion mit wasserstoffabgebenden Mitteln, z.B. Hydrazinen, wird durch die oben genannten Hydrierkatalysatoren, z.B. Raney-Nickel, Palladium-Kohle oder Platin beschleunigt. Die elektrolytische Reduktion der Nitrogruppen zum Amin wird an Kathoden aus Metallen mit grosser Ueberspannung, wie Blei, Zinn, Nickel, Kupfer oder Zink durchgeführt. Die Elektrolyse erfolgt meist in schwefelsaurer oder salzsaurer Lösung.

Die oben erwähnten Reduktionsmittel werden zumindest in äquimolarer Menge, bevorzugt im Ueberschuss, zugesetzt. Die Zugabe eines Ueberschusses an Reduktionsmittel soll die Bildung von Zwischenprodukten, z.B. Nitroso- oder Hydroxyamino-Verbindungen, unterbinden.

Die Reduktion wird vorzugsweise in einem Lösungsmittel durchgeführt, z.B. einem Niederalkanol, z.B. Methanol oder Aethanol, einem Niederalkoxyniederalkanol, z.B. 2-Methoxy- oder 2-Aethoxyäthanol, einer Niederalkancarbonsäure oder einem Ester davon, z.B. Essigsäure und Essigsäureäthylester, sowie einem Aether, z.B. Diäthyläther, Tetrahydrofuran oder Dioxan.

Um die Löslichkeit vor allem der salzartigen Reduktionsmittel im Reaktionsgemisch zu erhöhen, kann zum Reaktionsgemisch nach Bedarf Wasser hinzugesetzt werden. Man arbeitet gewöhnlicherweise bei Temperaturen von etwa −20°C bis etwa 100°C, wobei bei Verwendung von stark reaktionsfähigen Aktivierungsmitteln die Reaktion auch bei tieferen Temperaturen durchgeführt werden kann.

In einer Verbindung der Formel II wird eine austauschbare Gruppe X, z.B. Halogen, z.B. Chlor, Brom oder Jod, mit einer die Gruppe —N($R_2$)($R_3$) liefernden Verbindung, z.B. der Formel H—N($R_2$)($R_3$) oder einer Metallverbindung davon, wie Ammoniak, einem Alkalimetallamid, z.B. Lithium-, Natrium- oder Kaliumamid, einem Niederalkylamin, z.B. Methylamin, einem Diniederalkylamin, z.B. Dimethylamin, einem Säureamid, worin ein Wasserstoff der Amidgruppe durch ein Alkalimetall, z.B. Lithium, ersetzt ist, z.B. $R^a$—CO—$NR_3Li$, in die Gruppe —N($R_2$)($R_3$) umgewandelt.

Die Umsetzung einer Verbindung der Formel II, worin X Halogen, z.B. Chlor, bedeutet, mit einer die Gruppe —N($R_2$)($R_3$) liefernden Verbindung, z.B. mit Ammoniak, erfolgt vorzugsweise in Gegenwart eines Katalysators, z.B. Kupfer(I)-oxid oder -(II)-oxid, Kupfer (I)- oder -(II)-chlorid oder Kupfersulfat. Man arbeitet in einer konzentrierten wässrigen Ammoniaklösung oder vorzugsweise in flüssigem Ammoniak und hält die in Houben-Weyl, Methoden der Organischen Chemi (im folgenden "Houben-Weyl" genannt), Vol. XI/1 "Stickstoffverbindungen", S. 63—67 angegebenen Reaktionsbedingungen ein, z.B. erhöhte Temperatur oberhalb 100° und erhöhter Druck.

Die Umsetzung einer Verbindung der Formel II, worin X Halogen, z.B. Chlor bedeutet, erfolgt vorzugsweise mit einem Alkalimetallamid, z.B. Lithium- oder Kaliumamid. Man gibt zweckmässigerweise das Amid in Form einer Suspension hinzu.

Als Lösungsmittel verwendet man vorzugsweise Benzol oder Toluol, und arbeitet unter Inertgas-, z.B. Stickstoffatmosphäre. Die Umsetzung erfolgt am besten bei erhöhter Temperatur, z.B. bei der Siedetemperatur des Reaktionsgemisches, analog den in Houben-Weyl, Vol. XI/1 "Stickstoffverbindungen" auf S. 74—79 für aromatische Halogenverbindungen beschriebenen Reaktionsbedingungen.

In einer Verbindung der Formel II lässt sich eine derivatisierte Carboxylgruppe X, z.B. Carbamoyl oder Azidocarbonyl, unter der für Umsetzungen bzw. Abbaureaktionen nach Hofmann (Carbamoyl) oder Curtius (Azidocarbonyl) bekannten Reaktionsbedingungen in die Aminogruppe unwandeln.

Die Umwandlung nach Hofmann der Carbamoyl-Verbindung (Carbonsäureamid) der Formel II mit freiem Halogen, z.B. Brom, in die Aminoverbindung der Formel I erfolgt unter alkalischen Bedingungen.

Die Umwandlung nach Curtius der Azidocarbonyl-Verbindung der Formel II in die Aminoverbindung der Formel I erfolgt thermisch unter Zersetzung der Azidocarbonylgruppe.

Nach der Umlagerung verseift man unter sauren Bedingungen, z.B. in einer verdünnten, wässrigen Mineralsäure, z.B. in verdünnter Schwefelsäure.

Die Reaktionsbedingungen für den Hofmann- und Curtius-Abbau sind in dem Uebersichtsartikel von P. A. Smith, Org. Reactions 3, 363 (1946) beschrieben.

Geschützte Aminogruppen, aus welcher man die Schutzgruppe abspalten und durch Wasserstoff ersetzen kann sind beispielsweise in McOmie, "Protective Groups in Organic Chemistry", Plenum Press, London und New York 1973, in "The Peptides", Vol. I, Schröder und Lubke, Academic Press, London und New York 1965, sowie in Houben-Weyl, Vol. 15/1, Georg Thieme Verlag Stuttgart 1974, beschrieben.

Als Schutzgruppen sind acidolytisch abspaltbare Gruppen bevorzugt, beispielsweise Niederalkoxycarbonyl, z.B. tert.-Butoxycarbonyl (BOC) oder 2-Halogenniederalkoxycarbonyl, z.B. 2-Jodäthoxy- oder 2,2,2-Trichloräthoxycarbonyl.

Ferner kann man aber auch reduktiv oder unter milden Bedingungen basisch abspaltbare Amino-Schutzgruppen verwenden, z.B. insbesondere die Benzyloxycarbonyl-Gruppe oder eine Benzyloxycarbonyl-Gruppe, worin der Phenylrest durch Halogenatome, Nitrogruppen und/oder Niederalkoxygruppen substituiert ist, z.B. die p-Chlor-, p-Nitro- oder p-Methoxybenzyloxycarbonyl-Gruppe.

Die Abspaltung der Schutzgruppe erfolgt in der allgemein bekannten Weise; die saure Hydrolyse (Acidolyse) wird z.B. mittels Trifluoressigsäure durchgeführt. Die reduktiv abspaltbaren Gruppen, insbesondere solche, die Benzylreste enthalten, werden vorzugsweise hydrogenolytisch, z.B. durch Hydrierung unter Palladium-Katalyse, entfernt.

Verfahren b)

In einer Verbindung der Formel III sind die Gruppen $Y_1$ und $Y_2$ unabhängig voneinander, wie bereits gesagt Hydroxy, Halogen, z.B. Chlor, Brom oder Jod, Niederalkoxy, Silyloxy oder Sulfonyloxy.

Niederalkoxy $Y_1$ oder $Y_2$ ist beispielsweise Methoxy, Aethoxy, n-Propoxy, verzweigtes Niederalkoxy, z.B. tert-Butoxy, oder substituiertes Niederalkoxy, z.B. Benzyloxy, 4-Nitrobenzyloxy oder Diphenylmethoxy.

Silyloxy $Y_1$ oder $Y_2$ ist beispielsweise Triniederalkylsilyloxy, z.B. Trimethylsilyloxy.

Sulfonyloxy $Y_1$ oder $Y_2$ ist beispielsweise Niederalkansulfonyloxy, z.B. Methansulfonyloxy, Benzolsulfonyloxy oder p-Toluolsulfonyloxy.

Eine die Gruppe >N—$R_1$ liefernde Verbindung ist beispielsweise ein Alkalimetallamid, z.B. Natrium- oder Kaliumamid, Ammoniak ($R_1$ = Wasserstoff), ein Niederalkylamin, z.B. Methylamin ($R_1$ = Niederalkyl), ein Kohlensäureamid, z.B. Harnstoff oder 1,3-Dimethylharnstoff, oder ein Niederalkancarbonsäureamid, z.B. Formamid, N-Methylformamid, Acetamid oder N-Methylacetamid.

Die Umsetzung mit einer die Gruppe >N—$R_1$ liefernden Verbindung, z.B. mit Ammoniak oder mit Methylamin, kann stufenweise erfolgen. Beispielsweise kann man zunächst eine Verbindung der Formel III

erhalten, worin eine der Abgangsgruppen $Y_1$ und $Y_2$ durch —NH—$R_1$ ersetzt ist. Eine solche Verbindung, z.B. das Monoamid, lässt sich isolieren oder *in situ* durch Abspaltung von $HY_1$ oder $HY_2$ in eine Verbindung der Formel I überführen.

Eine Verbindung der Formel III, worin beispielsweise $Y_1$ und $Y_2$ Hydroxy bedeuten, kann man zunächst in ihr Anhydrid überführen, z.B. thermisch oder durch Dehydratisieren mit einem üblichen Dehydratisierungsmittel, z.B. Acetanhydrid oder Acetylchlorid. Dieses Anhydrid kann man isolieren oder *in situ* durch Umsetzung mit einer die Gruppe >N—$R_1$ liefernden Verbindung, z.B. der Formel $H_2N$—$R_1$, wie Ammoniak oder Methylamin, in eine Verbindung überführen, worin eine der Abgangsgruppen $Y_1$ oder $Y_2$ durch —NH—$R_1$ ersetzt ist. Ein solches Monoamid kann man anschliessend durch Abspalten von Wasser zu einer Verbindung der Formel I zyklisieren.

Bei der Zyklisierung werden insgesamt 2 Mol $HY_1$ oder $HY_2$, z.B. HCl oder HBr, freigesetzt, welche beispielsweise von einem eingesetzten Ueberschuss des >N—$R_1$-Lieferanten, z.B. Ammoniak oder Methylamin, gebunden werden.

Die Umsetzung wird bevorzugt in einem inerten polaren Lösungsmittel durchgeführt, z.B. in Benzol, Toluol, Xylol, Chlorobenzol, Dichlorobenzol, Methylenchlorid, Aether oder Methanol oder Gemischen davon. Die Reaktionstemperatur liegt zwischen −20° und ca. +180°C und bevorzugt zwischen +100° und +180°C. Setzt man ein Säurehalogenid der Formel III, z.B. das Säuredichlorid, mit Ammoniak um, arbeitet man vorzugsweise unter Kühlen, beispielsweise unter 0°.

Verfahren c)

Die Zyklisierung kann z.B. thermisch oder photochemisch erfolgen.

Der thermische Ringschluss der Acryloylacrylamide der Formel IV kann ohne Lösungsmittel durchgeführt werden, doch verwendet man dazu vorzugsweise Lösungen in hochsiedenden inerten Lösungmitteln, beispielsweise in Toluol, Xylol, z.B. 1,3-Xylol, Mesitylen, Chlorbenzol, Dichlorbenzol, z.B. 1,3-Dichlorbenzol, Nitrobenzol, Decalin, Hexachlorbutadien oder dergleichen. Die zu zyklisierende Verbindung der Formel IV wird in einer Konzentration von ca. 1 M bis ca. $10^{-6}$ M, bevorzugt in einer Konzentration von 1 M bis $10^{-2}$ M im Lösungsmittel gelöst und in einem offenen Gefäss gewünschtenfalls unter einem Schutzgas, z.B. Stickstoff oder Argon, oder in einem Druckgefäss verschlossen auf Temperaturen von ca. 100°C bis ca. 250°C, bevorzugt 130°C bis 180°C während ca. 1 bis ca. 10 Std., bevorzugt 2 bis 5 Std. erhitzt. Wird ohne Lösungsmittel gearbeitet, so wird das Acryloylacrylamid der Formel IV gewünschtenfalls unter vermindertem Druck verdampft, in einem niederigsiedenden inerten Lösungsmittel versprüht oder in pulverisierter Form in einen auf ca. 200°C bis ca. 500°C geheizten Reaktor eingebracht und gegebenenfalls mit einem inerten Trägergas, wie Stickstoff oder Argon, durch den geheizten Reaktor durchgeleitet.

Acryloylacrylamide der Formel IV können auch photochemisch zu Verbindungen der Formel I zyklisiert werden, insbesondere, wenn $R_4$ verschieden von Wasserstoff ist. Dazu werden die Verbindungen der Formel IV zusammen mit einem Triplett-Sensibilisator, beispielsweise einem Arylketon, z.B. Benzophenon oder Acetophenon, in einem photochemisch inerten Lösungsmittel, beispielsweise in Methylenchlorid, Diäthyläther, n-Pentan, n-Hexan, Cyclohexan, Benzol, Toluol oder dergleichen in einer Konzentration von ca. 1 M bis ca. $10^{-6}$ M gelöst und bei Temperaturen von ca. −30°C bis ca. +50°C, bevorzugt bei 0°C bis 30°C während ca. 1 bis ca. 10 Std., bevorzugt 2 bis 5 Std. mit Ultraviolett-Licht einer Wellenlänge von über 300 nm, beispielsweise mit einer Quecksilber-Hockdruck- oder Mitteldrucklampe in Pyrexglas® oder mit einem anderen Filter, der unter 300 nm lichtundurchlässig ist, bestrahlt. Vorzugsweise wird anstelle eines photochemisch inerten Lösungsmittels und eines Arylketons als Triplett-Sensibilisator ein Diniederalkylketon, z.B. Aceton, 2-Butanon oder 3-Pentanon, oder ein Cycloalkanon, z.B. Cyclohexanon, als Lösungsmittel und Sensibilisator für den photochemischen Ringschluss verwendet.

Zur Unterdrückung von unerwünschten Polymerisationsreaktionen wird den Lösungen der Verbindungen IV beim thermischen oder photochemischen Ringschluss vorzugsweise ein Radikalfänger, beispielsweise Hydrochinon,2,6-Di-tert-butyl-4-methylphenol oder Bis-(3-tert-butyl-4-hydroxy-5-methylphenyl)sulfid, in Konzentration von ca. 0,01% bis ca. 5% zugegeben.

Verfahren d)

In einer Verbindung der Formel V oder VI ist die Abgangsgruppe $Y_3$, wie bereits gesagt, Halogen, z.B. Chlor, Brom oder Jod, oder Sulfonyloxy, beispielsweise Niederalkansulfonyloxy, z.B. Methansulfonyloxy, oder Arylsulfonyloxy, z.B. Benzolsulfonyloxy oder p-Toluolsulfonyloxy.

Für die Zyklisierung geeignete Basen sind insbesondere nicht-nukleophile Basen. Beispiele für solche Basen sind Alkalimetallsalze, z.B. Lithium-, Natrium- oder Kaliumsalze, von sterisch gehinderten sekundären Aminen, z.B. verzweigten Diniederalkylaminen, Dicycloalkylaminen, verzweigten Niederalkyl-Cyclohexylaminen, Bis-(triniederalkylsilyl)aminen oder dergleichen, beispielsweise Lithiumdiisopropylamid, Lithiumdicyclohexylamid, Natrium- oder Kalium-bis-(trimethylsilyl)amid oder auch Lithium-2,2,6,6-tetramethylpiperidid. Geeignete Basen sind ferner Alkalimetallhydride, beispielsweise Kaliumhydrid, oder verzweigte Niederalkyllithium-Verbindungen, beispielsweise tert-Butyllithium.

Als Lösungsmittel verwendet man vorzugsweise einen Aether, beispielsweise Tetrahydrofuran oder Dimethoxyäthan, dem gegebenenfalls Hexamethylphosphorsäuretriamid zugesetzt wird. Die Reaktion erfolgt am besten unter Inertgas-, z.B. Stickstoff- oder Argonatmosphäre bei Temperaturen von ca. −80°C bis ca. +30°C, beispielsweise um −30°C.

Nachoperationen:

In einer Verbindung der Formel I können die Substituenten $R_1$, $R_2$, $R_3$ und $R_4$ im Rahmen ihrer Bedeutungen in andere Substituenten $R_1$, $R_2$, $R_3$ und $R_4$ überführt werden. So kann eine freie Aminogruppe in eine Acylaminogruppe umgewandelt werden, worin $R_2$ Acyl und $R_3$ Wasserstoff oder Niederalkyl bedeuten. Diese nachträglichen Operationen werden in an sich bekannter Weise durchgeführt, beispielsweise wie folgt:

Acylierung der Aminogruppe am Phenylring:

Wenn in einer erhältlichen Verbindung der Formel I $R_2$ Wasserstoff, $R_3$ Wasserstoff oder Niederalkyl und $R_1$ Wasserstoff oder ein Kohlenwasserstoff- rest sind, kann die freie Aminogruppe am Phenylring in an sich bekannter Weise durch eine Acylgruppe $R_2$ substituiert werden. Wenn $R_1$ Wasserstoff ist, kann der Stickstoff in Stellung 3 des Bicyclus (Imidstickstoff) gewünschtenfalls durch eine der weiter vorn unter Verfahren a) genannten Aminoschutzgruppen oder eine andere Aminoschutzgruppe, wie Niederalkanoyl, z.B. Acetyl, Niederalkenoyl, z.B. Methacryloyl, oder 1-Niederalkoxycarbonyl-1-alkenyl, z.B. 1-Methoxycarbonyl-1-vinyl, geschützt werden.

Diese Substitution kann beispielsweise durch Acylierung mit einem den entsprechenden Acylrest $R_2$ einführenden geeigneten Acylierungsmittel erfolgen.

Die Aminogruppe am Phenylring liegt in freier Form oder in reaktionsfähiger, d.h. die Acylierung erlaubender, beispielsweise durch Silylreste, geschützter Form vor.

Wird die Aminogruppe am Phenylring in einer Verbindung der Formel I durch einen Acylrest $R^a$—$SO_2$— substituiert, verwendet man als Acylierungsmittel beispielsweise die entsprechende Sulfonsäure oder ein reaktionsfähiges, funktionelles Derivat davon, in erster Linie ein Anhydrid dvaon, z.B. ein gemischtes Anhydrid. Ein gemischtes Anhydrid einer Sulfonsäure wird beispielsweise durch Kondensation mit einer anorganischen Säure, z.B. einer Halogenwasserstoffsäure, z.B. Chlorwasserstoff, gebildet und ist beispielsweise das entsprechende Sulfonsäurehalogenid, z.B. das Sulfonsäurechlorid oder -bromid.

Wird die Aminogruppe durch eine Acylgruppe $R^b$—CO— substituiert, verwendet man als Acylierungsmittel beispielsweise die entsprechende Carbonsäure selbst oder ein reaktionsfähiges, funktionelles Derivat davon.

Ein reaktionsfähiges, d.h. die Carboxamid-Funktion bildendes, funktionelles Derivat einer Carbonsäure ist ein Anhydrid dieser Carbonsäure, vorzugsweise ein gemischtes Anhydrid. Ein gemischtes Anhydrid wird beispielsweise durch Kondensation mit einer anderen Säure, z.B. einer anorganischen Säure, z.B. einer Halogenwasserstoffsäure, gebildet und ist beispielsweise das entsprechende Carbonsäurehalogenid, z.B. das Carbonsäurechlorid oder -bromid. Ein reaktionsfähiges, funktionelles Derivat einer Carbonsäure der Formel III wird ausserdem durch Kondensation mit einem Niederalkylhalbester der Kohlensäure, z.B. dem Aethyl- oder Isobutylhalbester der Kohlensäure, gebildet.

Wird die Aminogruppe am Phenylring durch einen Acylrest R mit den Bedeutungen $R^a$—O—CO—, $(R^b)(R^b)$N—CO— oder $(R^b)(R^b)$N—$SO_2$— substituiert, verwendet man als Acylierungsmittel ein reaktionsfähiges Derivat des entsprechenden Kohlensäurehalbesters, der entsprechenden Carbaminsäure oder Amidosulfonsäure. Solche reaktionsfähigen Derivate sind beispielsweise Anhydride, z.B. gemischte Anhydride, welche durch Kondensation mit anorganischen Säuren wie Halogenwasserstoffsäuren, z.B. Chlorwasserstoff, gebildet werden, oder sind bei Verwendung einer Carbaminsäure auch innere Anhydride, z.B. Isocyanate.

Die Acylierungsreaktionen werden bevorzugt in Anwesenheit eines geeigneten säurebindenden Mittels, beispielsweise einer geeigneten organischen Base, durchgeführt. Eine geeignete organische Base ist beispielsweise ein Amin, z.B. ein tertiäres Amin, z.B. Triniederalkylamin, z.B. Trimethylamin oder Triäthylamin, ein cyclisches tertiäres Amin, z.B. N-Methylmorpholin, ein bicyclisches Amidin, z.B. ein Diazabicycloalken, z.B. 1,5-Diazabicyclo[4.3.0]non-5-en oder 1,8-Diazabicyclo[5.4.0]undec-7-en (DBU), oder eine Base vom Pyridin-Typ, z.B. Pyridin oder 4-Dimethylaminopyridin. Ein geeignetes säurebindendes Mittel ist ferner eine anorganische Base, beispielsweise ein Alkalimetall- oder Erdalkalimetallhydroxid, z.B. Natrium-, Kalium- oder Calciumhydroxid.

Die Acylierungsreaktionen werden vorzugsweise in einem inerten, vorzugsweise wasserfreien, Lösungsmittel oder Lösungsmittelgemisch vorgenommen, beispielsweise in Dimethylformamid, Methylenchlorid, Tetrachlorkohlenstoff, Chlorbenzol, Aceton, Tetrahydrofuran, Essigsäureäthylester, oder Acetonitril, oder in Mischungen davon, gegebenenfalls bei erniedrigter oder erhöhter Temperatur, z.B. in einem Temperaturbereich von etwa −40° bis etwa +100°C, bevorzugt von etwa −10°C bis etwa +50°C, und gegebenenfalls unter Inertgas-, z.B. Stickstoffatmosphäre.

Die Acylierung der freien Aminogruppe am Phenylring kann sowohl im Endprodukt der Formel I als auch in den Zwischenprodukten der Formeln II, III und IV nach der weiter vorn angegebenen Methode erfolgen.

Substitution der Aminogruppe am Phenylring durch Sulfo:

Wenn in einer erhältlichen Verbindung der Formel I $R_2$ Wasserstoff, $R_3$ Wasserstoff oder Niederalkyl und $R_1$ Wasserstoff oder ein Kohlenwasserstoffrest sind, kann die freie Aminogruppe am Phenylring in an sich bekannter Weise durch Sulfo substituiert werden.

Dies kann beispielsweise durch Reaktion einer Aminophenylverbindung der Formel I mit Triäthylamin

in einem Schwefeltrioxid-Komplex erfolgen.

Damit in Verbindungen der Formel I, worin $R_1$, Wasserstoff ist, der Stickstoff in Stellung 3 des Bicyclus (Imidstickstoff) nicht durch Sulfo substituiert wird, ist dieser gewünschtenfalls durch eine der weiter vorn genannten Aminoschutzgruppen zu schützen.

Alkylierung der Aminogruppe am Phenylring:

Wenn in einer erhältlichen Verbindung der Formel I $R_2$ und $R_3$ Wasserstoff bedeuten, kann man die freie Aminogruppe am Phenylring durch zwei Aequivalente eines den Niederalkylrest einführenden, geeigneten Alkylierungsmittels, z.B. eines Alkylhalogenids, z.B. Methylbromid, zur Diniederalkyl-substituierten Aminogruppe ($R_2$ und $R_3$ = Niederalkyl) substituieren. Je nach Reaktionsbedingungen, z.B. auch bei Verwendung von weniger als zwei Aequivalenten des Alkylierungsmittels, entstehen Gemische von Verbindungen mit Diniederalkyl-substituierter Aminogruppe, mit Mononiederalkyl-substituierter Aminogruppe ($R_2$ oder $R_3$ = Niederalkyl) oder mit unsubstituierter Aminogruppe. Diese Gemische lassen sich in an sich bekannter Weise, beispielsweise durch fraktionierte Kristallisation oder durch chromatographische Methoden, trennen.

Die freie Aminogruppe am Phenylring kann man auch durch eine der vorn genannten, üblichen Aminoschutzgruppen, z.B. durch tert-Butoxycarbonyl, schützen und nach anschliessender Metallierung der so geschützten Aminogruppe mit einem geeigneten Metallierungsreagenz, gefolgt von einer dem Niederalkylrest $R_2$ oder $R_3$ entsprechenden, reaktionsfähigen, alkylierenden Verbindung, alkylieren. Nach Abspaltung der Aminoschutzgruppen erhält man eine Mononiederalkyl-substituierte Aminogruppe ($R_2$ = H und $R_3$ = Niederalkyl oder $R_2$ = Niederalkyl und $R_3$ = H).

Geeignete Metallierungsreagenzien sind beispielsweise Lithiumdiisopropylamid oder Butyllithium. Eine dem Rest $R_3$ entsprechende reaktionsfähige Verbindung ist beispielsweise eine Verbindung der Formel $R_2$—X oder $R_3$—X, worin X eine nukleofuge Abgangsgruppe, beispielsweise ein Halogenatom, z.B. Chlor, Brom oder Jod, oder eine Sulfonyloxygruppe, z.B. Methansulfonyloxy oder p-Toluolsulfonyloxy, darstellt.

Soll der Imidstickstoff in Verbindungen mit $R_1$ = Wasserstoff nicht alkyliert werden, ist dieser gegebenenfalls durch eine der weiter vorn genannten Schutzgruppen zu schützen.

Weitere Nachoperationen:

Die Trennung von erfindungsgemässen erhältlichen Isomerengemischen in reine Isomere erfolgt in an sich bekannter Weise, z.B. nach physikalischen oder chemischen Methoden, beispielsweise durch fraktionierte Kristallisation. Man kann aber auch chromatographische Methoden, z.B. Fest- Flüssig-Chromatographie, verwenden. Leichtflüchtige Isomerengemische können auch destillativ oder chromatographisch getrennt werden.

Man erhält Säureadditionssalze in üblicher Weise, z.B. durch Behandeln mit einer Säure, oder einem geeigneten Anionenaustauscherreagenz.

Das Verfahren umfasst auch diejenigen Ausführungsformen, wonach als Zwischenprodukte anfallende Verbindungen als Ausgangsstoffe verwendet und die restlichen Verfahrensschritte mit diesen durchgeführt werden, oder das Verfahren auf irgendeiner Stufe abgebrochen wird; ferner können Ausgangstoffe in Form von Derivaten verwendet oder während der Reaktion gebildet werden.

Vorzugsweise werden solche Ausgangsstoffe verwendet und die Reaktionsbedingungen so gewählt, dass man zu den vorstehend als besonders bevorzugt aufgeführten Verbindungen gelangt.

Ausgangsmaterialien

Die im Verfahren zur Herstellung der Verbindungen der Formel I der vorliegenden Erfindung verwendeten Ausgangsmaterialien und Zwischenprodukte sind bekannt oder können, falls sie neu sind, in an sich bekannter Weise hergestellt werden. Neue Zwischenprodukte und Verfahren zu ihrer Herstellung sind ebenfalls Gegenstand der vorliegenden Erfindung.

Verbindungen der Formel II, worin $R_1$ und $R_4$ die unter Formel I genannte Bedeutung haben und worin X eine Stickstoff enthaltende reduzierbare Gruppe, z.B. die Nitro-, Nitroso-, Hydroxyamino- oder Azidogruppe, Halogen, z.B. Chlor, Brom oder Jod, die Carbamoylgruppe Azidocarbonylgruppe oder eine geschützte Aminogruppe bedeutet, sind neu und ebenfalls Gegenstand der vorliegenden Erfindung. Bevorzugte Ausgangsmaterialien der Formel II sind Verbindungen, worin X eine Nitrogruppe darstellt. Man

kann Verbindungen der Formel II oder Salze davon in an sich bekannter Weise herstellen, indem man beispielsweise

e) eine Verbindung der Formel

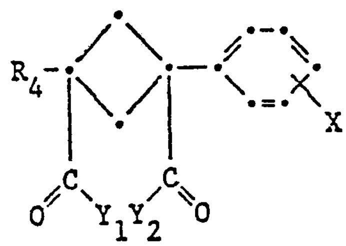

(VII),

worin $R_4$ die unter Formel I genannte Bedeutung hat und $Y_1$ und $Y_2$ unabhängig voneinander Hydroxy, Halogen, Niederalkoxy, Silyloxy oder Sulfonyloxy oder zusammen Sauerstoff und X' eine der unter Formel II oben angegebenen Gruppen X oder Carboxyl darstellen, oder ein Salz davon durch Umsetzung mit einer die Gruppe

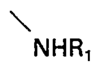

liefernden Verbindung zyklisiert, oder

f) eine Verbindung der Formel

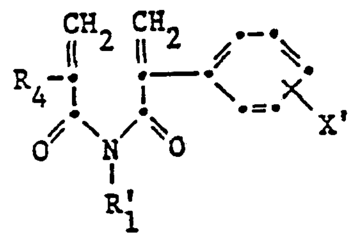

(VIII),

worin $R_1'$ eine Gruppe $R_1$ oder eine Aminoschutzgruppe und X' eine Gruppe X oder Carboxyl darstellen und $R_1$, $R_4$ und X die oben genannten Bedeutungen haben, oder ein Salz davon thermisch in Abwesenheit bzw. Anwesenheit eines inerten Lösungsmittels oder auch photochemisch zusammen mit einem Triplett-Sensibilisatoren in einem photochemisch inerten Lösungsmittel zyklisiert

g) eine Verbindung der Formel

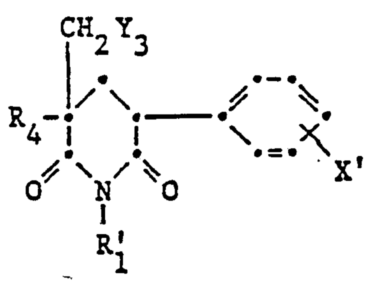

oder

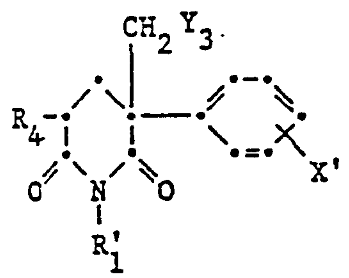

(IX) (X)

worin $R_1'$ eine Gruppe $R_1$ oder eine Aminoschutzgruppe, X' eine Gruppe X oder Carboxyl und $Y_3$ Halogen oder Sulfonyloxy bedeutet in Gegenwart einer nicht-nukleophilen Base zyklisiert und in einer erhältlichen Verbindung die Carboxylgruppe X' in Carbamoyl oder Azidocarbonyl überführt und/oder die Aminoschutzgruppe $R_1'$ abspaltet und gewünschtenfalls ein erhältliches Salz in die freie Verbindung oder in ein anderes Salz überführt und/oder eine erhältliche freie Verbindung in ein Salz überführt und/oder ein erhältliches Gemisch von Isomeren in die einzelnen Isomere auftrennt.

Das Verfahren 3) lässt sich analog den weiter vorn unter Verfahren b), das Verfahren f) analog den unter Verfahren c) und das Verfahren g) analog den unter Verfahren d) genannten Reaktionsbedingungen durchführen. Die Umwandlung der Carboxylgruppe X' in einer Verbindung der Formel II in eine Azidocarbonyl- bzw. Carbamoylgruppe lässt sich anhand der im Organikum, VEB Deutscher Verlag der

Wissenschaften 15. Auflage 1976, für aromatische Carboxylgruppen beschriebenen Derivatisierungsverfahren durchführen. Die Abspaltung von Aminoschutzgruppen $R_1'$ wird analog der unter Verfahren a) beschriebenen Art und Weise durchgeführt.

Geeignete Aminoschutzgruppen $R_1'$ und ihre Abspaltung sind weiter vorn unter Verfahren a) beschrieben. Ebenfalls geeignet als Aminoschutzgruppen $R_1'$ sind beispielsweise Niederalkanoyl, z.B. Acetyl oder Propionyl, oder Niederalkenoyl, z.B. Acryloyl oder insbesondere Methacryloyl. Diese Schutzgruppen werden acidolytisch abgespalten, beispielsweise mittels Essigsäure, Dichloressigsäure, Trifluoressigsäure oder verdünnter Salzsäure und dergleichen. Weiter geeignet als Aminoschutzgruppe $R_1'$ ist beispielsweise 1-Niederalkoxycarbonyl-1-alkenyl, z.B. 1-Methoxycarbonyl-1-vinyl. Diese Schutzgruppen werden oxidativ abgespalten, beispielsweise mittels Kaliumpermanganat, Natriumperjodat und einer katalytischen Menge Osmiumtetroxid, oder Ozon.

Verbindungen der Formel III und der Formel VII, worin $R_2$, $R_3$ und $R_4$ die unter Formel I genannte Bedeutung haben, $Y_1$ und $Y_2$ die unter Formel III definierten Gruppen bedeuten und X' eine Stickstoff enthaltende, reduzierbare Gruppe, Halogen, Carbamoyl, Azidocarbonyl, eine geschützte Aminogruppe oder Carboxyl darstellen, können beispielsweise hergestellt werden, indem man Verbindungen der Formel

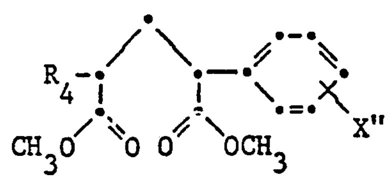

(XI)

worin $R_4$ die unter Formel I genannte Bedeutung hat und X'' die $—N(R_2)(R_3)$-Gruppe, eine Stickstoff enthaltende, reduzierbare Gruppe, Halogen, Carbamoyl, Azidocarbonyl, eine geschützte Aminogruppe oder Carboxyl darstellt, analog der von P. M. Warner et al. in J. Org. Chem. *46*, 4795 (1981) beschriebenen Weise mit Methylenjodid und Lithiumdiisopropylamid in α-Stellung zu einer Esterfunktion alkyliert und dann analog der in Verfahren d) beschriebenen Weise durch Abspaltung von Jodwasserstoff mit Base zyklisiert und schliesslich gegebenenfalls die Methoxygruppe der Esterfunktionen in eine andere Abgangsgruppe $Y_1$ bzw. $Y_2$ umwandelt.

Methoden zur Umwandlung einer Methoxygruppe eines Esters in eine Abgangsgruppe, beispielsweise in Hydroxy, Chlor, Brom, Jod oder Niederalkoxy oder in ein Anhydrid, sind beschrieben in Houben-Weyl, Vol. VIII, "Sauerstoffverbindungen III", Kapitel 4 und 5.

Glutarester-Derivate der Formel XI können, soweit sie nicht bekannt sind, analog der in U.S. Patent 2 824 120 beschriebenen Methode aus substituierten Phenylessigsäureestern und gegebenenfalls α-substituierten Acrylestern gewonnen werden.

Verbindungen der Formel IV und der Formel VIII, worin $R_1$, $R_2$, $R_3$ und $R_4$ die unter Formel I genannte Bedeutung haben, $R_1'$ eine Gruppe $R_1$ oder eine Aminoschutzgruppe und X' eine in die $—N(R_2)(R_3)$-Gruppe überführbare Gruppe oder Carboxyl darstellen, können analog der von A. Alder et al. in Helv. Chim. Acta *65*, 2405 (1982) und J. Am. Chem. Soc. *105*, 6712 (1983) beschriebenen Art und Weise aus im Phenylring substituierten α-Phenylacrylsäuren und gegebenenfalls in α-Stellung und/oder am Stickstoff substituierten Acrylamiden gewonnen werden.

Verbindungen der Formel V und VI oder der Formel IX und X, worin $R_1$, $R_2$, $R_3$ und $R_4$ die unter Formel I genannte Bedeutung haben, $R_1'$ eine Gruppe $R_1$ oder eine Aminoschutzgruppe und X' eine Stickstoff enthaltende, reduzierbare Gruppe, Halogen, Carbamoyl, Azidocarbonyl, eine geschützte Aminogruppe oder Carboxyl darstellen, können beispielsweise erhalten werden, indem man Verbindungen der Formel

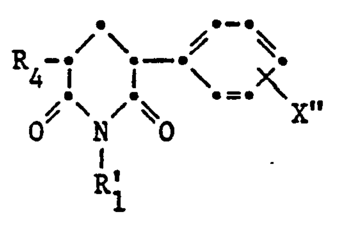

(XII)

worin $R_1'$ und $R_4$ die obengenannten Bedeutungen haben und X'' die $—N(R_2)(R_3)$-Gruppe, eine Stickstoff enthaltende, reduzierbare Gruppe, Halogen, Carbamoyl, Azidocarbonyl, eine geschützte Aminogruppe oder Carboxyl darstellt, analog der von P. M. Warner et al. in J. Org. Chem. *46*, 4795 (1981) beschriebenen Weise mit Methylenjodid und Lithiumdiisopropylamid in α-Stellung zu einer Carbonylfunktion jod- alkyliert oder mit Formaldehyd und einer nicht-nukleophilen Base entsprechend hydroxyalkyliert und die Hydroxygruppe anschliessend in an sich bekannter Weise in Halogen oder eine Sulfonyloxygruppe überführt.

Das Verhältnis der bei dieser Reaktion gebildeten Isomeren der Formel V und VI bzw. IX und X wird dabei von der Art der Substituenten $R_4$ und X'' beeinflusst, ist jedoch nicht von Belang, da in der

nachfolgenden Zyklisierung gemäss Verfahren d) bzw. g) jeweils einheitliche Verbindungen der Formel I bzw. II entstehen.

Glutarimid-Derivate der Formel XII können, soweit sie nicht bekannt sind, aus dem entsprechenden Glutarester-Derivat der Formel XI durch Reaktion mit einer den Rest >N—R'₁ einführenden Verbindung analog dem Verfahren b) erhalten oder durch Hydrierung von entsprechend substituierten 2-Hydroxy-6-oxo-1,6-dihydro-pyridinen der Formel

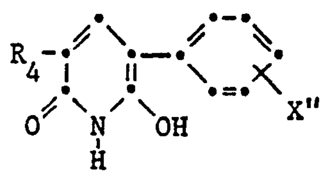

(XIII)

oder einer tautomeren Verbindung davon und gegebenenfalls anschliessender Alkylierung oder Acylierung des Imidstickstoffs mit einer den Rest $R_1'$ einführenden Verbindung hergestellt werden.

Verbindungen der Formel XIII sind bekannt oder können in der von E. Ziegler et al. in Z. Naturforsch. *33b*, 1550 (1978) beschriebenen Art und Weise gewonnen werden.

Die folgenden Beispiele dienen zur Illustration der Erfindung, beschränken sie hingegen in keiner Art und Weise. Temperaturen sind in Grad Celsius angegeben.

Abkürzungen:
Min. = Minuten
Smp. = Schmelzpunkt
Std. = Stunden
IR = Infrarot

Die $R_f$-Werte werden auf Kieselgel-Dünnschichtplatten bestimmt. $R_f(CH_2Cl_2)$ bedeutet beispielsweise, dass der $R_f$-Wert im Laufmittel $CH_2Cl_2$(Methylenchlorid) ermittelt wird.

## Beispiel 1

a) 1-(4-Aminophenyl)-3-n-propyl-3-azabicyclo[3.1.1]heptan-2,4-dion

Eine Lösung von 3,5 g 1-(4-Nitrophenyl)-3-n-propyl-3-azabicyclo[3.1.1]heptan-2,4-dion in 70 ml Essigester wird mit 0,35 g 5%igem Palladium/Kohle-Katalysator versetzt und bei Normaldruck und 30—35° in Wasserstoffatmosphäre hydriert. Nach beendeter Wasserstoffaufnahme wird das Reaktionsgemisch mit 30 ml Methylenchlorid verdünnt und durch Filtration über HYFLO-Super-Cel® vom Katalysator befreit. Man dampft im Vakuum das Lösungsmittel ab, kristallisiert den Rückstand aus einem Essigester/n-Hexan-Gemisch um und erhält so die Titelverbindung als weisse Kristalle mit einem Smp. von 166—167°. IR-Spektrum in $CHCl_3$: 1685 und 1740 $cm^{-1}$.

### Herstellung des Ausgangsmaterials

b) 1-(4-Nitrophenyl)-3-n-propyl-3-azabicyclo[3.1.1]heptan-2,4-dion

Eine Lösung von 46,1 g 4-Aza-2-(4-nitrophenyl)-4-n-propyl-1,6-heptadien-3,5-dion und 0,5 g 2,6-Di-tert-butyl-p-kresol in 900 ml 1,3-Dichlorbenzol wird während 1½ Std. bei 170° gerührt. Nach dem Eindampfen wird der Rückstand aus einem Methylenchlorid-Diisopropyläther-Gemisch umkristallisiert, und man erhält so die Titelverbindung als blassgelbe Kristalle mit einem Smp. von 141—143°. Die eingedampfte Mutterlauge wird mit Methylenchlorid an Kieselgel 60 filtriert. Das so erhaltene Produkt ergibt nach dem Umkristallisieren aus einem Methylenchlorid/Diisopropyläther-Gemisch weisse Kristalle mit einem Smp. von 149—151°. IR-Spektrum in $CHCl_3$: 1350, 1685 und 1745 $cm^{-1}$.

c) 4-Aza-2-(4-nitrophenyl)-4-n-propyl-1,6-heptadien-3,5-dion

Zu einer gerührten Suspension von 115,8 g α-(4-Nitrophenyl)-acrylsäure in 5 ml Dimethylformamid und 2,5 l Methylenchlorid wird während 2 Std. bei Raumtemperatur eine Lösung von 76,2 g Oxalylchlorid in 500 ml Methylenchlorid getropft. Es wird nach beendeter Zugabe noch 2 Std. gerührt, bis die Gasentwicklung beendet ist. Die so erhaltene Lösung von 2-(4-Nitrophenyl)-acrylsäurechlorid wird auf 0° gekühlt und in eine auf 0—5° gekühlte Lösung von 67,8 g N-n-Propyl-acrylamid und 121 g Triäthylamin in 450 ml Methylenchlorid getropft. Nach beendeter Zugabe wird 1½ Std. bei Raumtemperatur nachgerührt. Nach dem Eindampfen wird der Rückstand mit 2 l Aether verrührt, abgenutscht und der Rückstand nochmals mit Methylenchlorid verrührt und wiederum abgenutscht. Das Aetherfiltrat wird im Vakuum zur Trockene eingedampft, und der Rückstand wird mit 3 l Hexan erwärmt, mit Aktivkohle versetzt und heiss filtriert. Man erhält nach dem Erkalten die Titelverbindung als blassrosa Kristalle mit einem Smp. von 68—69,5°.

Das Methylenchloridfiltrat wird auf ein Volumen von ca. 300 ml eingedampft, abgenutscht und an 1,5 kg Kieselgel 60 in Aether filtriert. Nach dem Eindampfen des Filtrates und Umkristallisieren aus Hexan erhält man ebenfalls kristalline Titelverbindung mit einem Smp. von 69—70°. Nochmalige Umkristallisation der vereinigten Produkte aus Hexan ergibt weisses kristallines Produkt mit einem Smp. von 71,5—72,5°.

Beispiel 2

a) 1-(4-Aminophenyl)-3-methyl-3-azabicyclo[3.1.1]heptan-2,4-dion

Analog Beispiel 1a werden 6,0 g 3-Methyl-1-(4-nitrophenyl)-3-azabicyclo[3.1.1]heptan-2,4-dion in 120 ml 2-Methoxyäthanol gelöst, in Gegenwart von 0,6 g 5%iger Palladium/Kohle hydriert und aufgearbeitet. Smp. 197—198° (aus Essigester). IR-Spektrum in $CHCl_3$: 1680 und 1740 $cm^{-1}$.

Herstellung des Ausgangsmaterials

b) 3-Methyl-1-(4-nitrophenyl)-3-azabicyclo[3.1.1]heptan-2,4-dion

Analog Beispiel 1b werden 37,1 g 4-Aza-4-methyl-2-(4-nitrophenyl)-1,6-heptadien-3,5-dion und 0,4 g 2,6-Di-tert-butyl-p-kresol in 370 ml 1,3-Dichlorbenzol bei 170° gerührt und aufgearbeitet. Smp. 185—188° (aus Methylenchlorid/Diisopropyläther). IR-Spektrum in $CHCl_3$: 1350, 1685 und 1740 $cm^{-1}$.

c) 4-Aza-4-methyl-2-(4-nitrophenyl)-1,6-heptadien-3,5-dion

Die Titelverbindung wird ausgehend von 115,8 g α-(4-Nitrophenyl)-acrylsäure, 76,2 g Oxalylchlorid und 51,1 g N-Methylacrylamid analog Beispiel 1c hergestellt. Das gelbe Oel wird roh weiterverarbeitet.

Beispiel 3

a) 1-(4-Aminophenyl)-3-isopropyl-3-azabicyclo[3.1.1]heptan-2,4-dion

Analog Beispiel 1a werden 2,3 g 3-Isopropyl-1-(4-nitrophenyl)-3-azabicyclo[3.1.1]heptan-2,4-dion in 50 ml Essigester gelöst, in Gegenwart von 0,25 g 5%iger Palladium/Kohle hydriert und aufgearbeitet. Smp. 191—201° (aus Essigester/Hexan). IR-Spektrum in $CHCl_3$: 1680 und 1740 $cm^{-1}$.

Herstellung des Ausgangsmaterials

b) 3-Isopropyl-1-(4-nitrophenyl)-3-azabicyclo[3.1.1]heptan-2,4-dion

Analog Beispiel 1b werden 10,2 g 4-Aza-4-isopropyl-2-(4-nitrophenyl)-1,6-heptadien-3,5-dion und 0,2 g 2,6-Di-tert-butyl-p-kresol in 100 ml 1,3-Dichlorbenzol bei 170° gerührt und aufgearbeitet. $R_f(CH_2Cl_2) = 0,26$. IR-Spektrum in $CHCl_3$: 1350, 1680 und 1740 $cm^{-1}$.

c) 4-Aza-4-isopropyl-2-(4-nitrophenyl)-1,6-heptadien-3,5-dion

Die Titelverbindung wird ausgehend von 44,6 g α-(4-Nitrophenyl)-acrylsäure, 29,8 g Oxalylchlorid und 21,8 g N-Isopropyl-acrylamid analog Beispiel 1c hergestellt; öliger Festkörper, $R_f(CH_2Cl_2) = 0,3$.

Beispiel 4

a) 1-(4-Aminophenyl)-3-neopentyl-3-azabicyclo[3.1.1]heptan-2,4-dion

Analog Beispiel 1a werden 4,1 g 3-Neopentyl-1-(4-nitrophenyl)-3-azabicyclo[3.1.1]heptan-2,4-dion in 80 ml Essigester gelöst, in Gegenwart von 0,4 g 5%iger Palladium/Kohle hydriert und aufgearbeitet. Smp. 141,5—143° (aus Diäthyläther). IR-Spektrum in $CHCl_3$: 1685 und 1740 $cm^{-1}$.

Herstellung des Ausgangsmaterials

b) 3-Neopentyl-1-(4-nitrophenyl)-3-azabicyclo[3.1.1]heptan-2,4-dion

Analog Beispiel 1b werden 12,1 g 4-Aza-4-neopentyl-2-(4-nitrophenyl)-1,6-heptadien-3,5-dion und 0,2 g 2,6-Di-tert-butyl-p-kresol in 120 ml 1,3-Dichlorbenzol bei 170° gerührt und aufgearbeitet. Man erhält die Titelverbindung nach einer Filtration an Kieselgel 60 mit Methylenchlorid als gelbe Kristalle mit einem Smp. von 175—182°, $R_f(\text{Aether}) = 0,56$. IR-Spektrum in $CHCl_3$: 1350, 1690 und 1745 $cm^{-1}$.

c) 4-Aza-4-neopentyl-2-(4-nitrophenyl)-1,6-heptadien-3,5-dion

Die Titelverbindung wird ausgehend von 45,9 g α-(4-Nitrophenyl)-acrylsäure, 30,2 g Oxalylchlorid und 28,0 g N-Neopentyl-acrylamid analog Beispiel 1c hergestellt. Gelbes Oel, $R_f(CH_2Cl_2) = 0,36$.

Alternative Herstellung des Ausgangsmaterials

d) 3-Neopentyl-1-(4-nitrophenyl)-3-azabicyclo[3.1.1]heptan-2,4-dion

Zu einer Suspension von 2,5 g 1-(4-Nitrophenyl)-1,3-cyclobutandicarbonsäureanhydrid in 6 ml Essigsäure werden bei Raumtemperatur 2,4 ml Neopentylamin zugetropft. Das Gemisch wird während 20 Std. bei 120° gerührt. Nach dem Eindampfen wird der Rückstand an Kieselgel mit einem 2:1 Aether/Hexan-Gemisch chromatographiert. Man erhält eine Fraktion, die mit der Titelverbindung von Beispiel 4b identisch ist.

e) 1-(4-Nitrophenyl)-1,3-cyclobutan-dicarbonsäureanhydrid

Ein Gemisch von 10 g cis-1-(4-Nitrophenyl)-1,3-cyclobutandicarbonsäure und 100 ml Essigsäureanhydrid wird während 2 Std. unter Rückfluss erwärmt. Das Reaktionsgemisch wird zur Trockene eingedampft, der Rückstand in Toluol suspendiert, abgenutscht, mit Aether gewaschen und über Nacht am Vakuum getrocknet. Man erhält so die Titelverbindung als graue Kristalle mit einem Smp. von 183—187°. IR-Spektrum (KBr-Pressling): 1355, 1780 und 1825 $cm^{-1}$.

f) Cis-1-(4-nitrophenyl)-1,3-cyclobutandicarbonsäure

Eine Suspension von 36,3 g 1-(4-Nitrophenyl)-3-n-propyl-3-azabicyclo[3.1.1]heptan-2,4-dion, 250 ml Essigsäure und 500 ml 50%iger Schwefelsäure wird während 20 Std. bei 140° gerührt. Nach dem Abkühlen wird auf Eis gegossen und viermal mit Aether extrahiert. Die Aetherauszüge werden zweimal mit wässriger Kochsalzlösung gewaschen, mit Magnesiumsulfat getrocknet und eingedampft. Der Rückstand wird mit Toluol versetzt und im Vakuum nochmals eingedampft. Nach der Umkristallisation aus Essigester erhält man weisse Kristalle mit einem Smp. von 218—219°.

Beispiel 5

a) 1-(4-Aminophenyl)-3-n-decyl-3-azabicyclo[3.1.1]heptan-2,4-dion

Analog Beispiel 1a werden 6,0 g 3-n-Decyl-1-(4-nitrophenyl)-3-azabicyclo[3.1.1]heptan-2,4-dion in 120 ml 2-Methoxyäthanol gelöst, in Gegenwart von 0,6 g 5%iger Palladium/Kohle hydriert und aufgearbeitet. Smp. 81,5—82,5° (aus Essigester/Hexan). IR-Spektrum in $CHCl_3$: 1680 und 1740 $cm^{-1}$.

Herstellung des Ausgangsmaterials

b) 3-n-Decyl-1-(4-nitrophenyl)-3-azabicyclo[3.1.1]heptan-2,4-dion

Analog Beispiel 1b werden 36,5 g 4-Aza-4-n-decyl-2-(4-nitrophenyl)-1,6-heptadien-3,5-dion und 0,4 g 2,6-Di-tert-butyl-p-kresol in 370 ml 1,3-Dichlorbenzol bei 170° gerührt und aufgearbeitet. Man erhält die Titelverbindung nach einer Filtration an Kieselgel 60 mit einem Aether/Hexangemisch 1:1 als gelbes Oel, $R_f$(Aether) = 0,54. IR-Spektrum in $CHCl_3$: 1350, 1685 und 1740 $cm^{-1}$.

c) 4-Aza-4-n-decyl-2-(4-nitrophenyl)-1,6-heptadien-3,5-dion

Die Titelverbindung wird ausgehend von 37,8 g α-(4-Nitrophenyl)-acrylsäure, 24,9 g Oxalylchlorid und 41,7 g N-n-Decyl-acrylamid analog Beispiel 1c hergestellt. Gelbes Oel.

Beispiel 6

a) 1-(4-Aminophenyl)-3-cyclohexylmethyl-3-azabicyclo[3.1.1]heptan-2,4-dion

Analog Beispiel 1a werden 6,0 g 3-Cyclohexylmethyl-1-(4-nitrophenyl)-3-azabicyclo[3.1.1]heptan-2,4-dion in 120 ml 2-Methoxyäthanol gelöst, in Gegenwart von 0,6 g 5%iger Palladium/Kohle hydriert und aufgearbeitet. Smp. 140—146° (aus Aether), $R_f$(Aether) = 0,25. IR-Spektrum in $CHCl_3$: 1685 und 1740 $cm^{-1}$.

Herstellung des Ausgangsmaterials

b) 3-Cyclohexylmethyl-1-(4-nitrophenyl)-3-azabicyclo[3.1.1]heptan-2,4-dion

Analog Beispiel 1b werden 22,3 g 4-Aza-4-cyclohexylmethyl-2-(4-nitrophenyl)-1,6-heptadien-3,5-dion und 0,3 g 2,6-Di-tert-butyl-p-kresol in 220 ml 1,3-Dichlorbenzol bei 170° gerührt und aufgearbeitet. Smp. 191—194° (aus Methylenchlorid/Diisopropyläther). IR-Spektrum in $CHCl_3$: 1350, 1685 und 1740 $cm^{-1}$.

c) 4-Aza-4-cyclohexylmethyl-2-(4-nitrophenyl)-1,6-heptadien-3,5-dion

Die Titelverbindung wird ausgehend von 47,9 g α-(4-Nitrophenyl)-acrylsäure, 31,5 g Oxalylchlorid und 34,7 g N-Cyclohexylmethyl-acrylamid analog Beispiel 1c hergestellt, hellgelbe Kristalle mit einem Smp. von 92—93°.

Beispiel 7

a) 1-(4-Aminophenyl)-3-cyclohexyl-3-azabicyclo[3.1.1]heptan-2,4-dion

Analog Beispiel la werden 6,0 g 3-Cyclohexyl-1-(4-nitrophenyl)-3-azabicyclo[3.1.3]heptan-2,4-dion in 120 ml 2-Methoxyäthanol gelöst, in Gegenwart von 0,6 g 5%iger Palladium/Kohle hydriert und aufgearbeitet. Smp. 139—140° (aus Aether). IR-Spektrum in $CHCl_3$: 1680 und 1735 $cm^{-1}$.

Herstellung des Ausgangsmaterials

b) 3-Cyclohexyl-1-(4-nitrophenyl)-3-azabicyclo[3.1.1]heptan-2,4-dion

Analog Beispiel 1b werden 34,6 g 4-Aza-4-cyclohexyl-2-(4-nitrophenyl)-1,6-heptadien-3,5-dion und 0,4 g 2,6 - Di - tert - butyl - p - kresol in 350 ml 1,3-Dichlorbenzol bei 170° gerührt und aufgearbeitet. Smp. 163—164° (aus Methylenchlorid/Diisopropyläther). IR-Spektrum in $CHCl_3$: 1350, 1690 und 1745 $cm^{-1}$.

c) 4-Aza-4-cyclohexyl-2-(4-nitrophenyl)-1,6-heptadien-3,5-dion

Die Titelverbindung wird ausgehend von 40,5 g α-(4-Nitrophenyl)acrylsäure, 26,7 g Oxalylchlorid und 26,8 g N-Cyclohexylacrylamid analog Beispiel 1c hergestellt. Smp. 73—74° (aux Hexan).

Beispiel 8

a) 1-(4-Aminophenyl)-3-(4-methoxybenzyl)-3-azabicyclo[3.1.1]heptan-2,4-dion

Analog Beispiel la werden 5,0 g 3-(4-Methoxybenzyl)-1-(4-nitrophenyl)-3-azabicyclo[3.1.1]heptan-2,4-dion in 100 ml Essigester gelöst, in Gegenwart von 1 g 5%iger Palladium/Kohle hydriert und aufgearbeitet. Smp. 147—147,5° (aus Aether). IR-Spektrum in $CHCl_3$: 1680 und 1735 $cm^{-1}$.

Herstellung des Ausgangsmaterials

b) 3-(4-Methoxybenzyl)-1-(4-nitrophenyl)-3-azabicyclo[3.1.1]heptan-2,4-dion

Analog Beispiel 1b werden 78,2 g 4-Aza-4-(4-methoxybenzyl)-2-(4-nitrophenyl)-1,6-heptadien-3,5-dion und 0,4 g 2,6-Di-tert-butyl-p-kresol in 700 ml 1,3-Dichlorbenzol bei 170° gerührt und aufgearbeitet. Smp. 146—147° (aus Toluol/Aether). IR-Spektrum in $CHCl_3$: 1350, 1685 und 1785 $cm^{-1}$.

c) 4-Aza-4-(4-methoxybenzyl)-2-(4-nitrophenyl)-1,6-heptadien-3,5-dion

Die Titelverbindung wird ausgehend von 106,1 g α-(4-Nitrophenyl)acrylsäure, 76,8 g Oxalylchlorid und 95,5 g N-(4-Methoxybenzyl)acrylamide analog Beispiel 1c hergestellt. Smp. 106,5—107° (aus Aether).

Beispiel 9

a) 1-(4-Aminophenyl)-3-azabicyclo[3.1.1]heptan-2,4-dion

Analog Beispiel la werden 5,0 g 1-(4-Nitrophenyl)-3-azabicyclo[3.1.1]heptan-2,4-dion in 250 ml 2-Methoxyäthanol in Gegenwart von 0,5 g 5%iger Palladium/Kohle bei 45° hydriert und aufgearbeitet. Man erhält die Titelverbindung, welche nach Umkristallisation aus 2-Methoxyäthanol bei 265° unter Zersetzung schmilzt. IR-Spektrum (KBr-Pressling): 1700 und 1735 $cm^{-1}$.

Herstellung des Ausgangsmaterials

b) 1-(4-Nitrophenyl)-3-azabicyclo[3.1.1]heptan-2,4-dion

Zu einer gerührten Lösung von 50 g 3-(4-Methoxybenzyl)-1-(4-nitrophenyl)-3-azabicyclo[3.1.1.]heptan-2,4-dion in 1,3 l Acetonitril wird bei Raumtemperatur eine Lösung von 283 g Cer(IV)ammoniumnitrat in 400 ml Wasser zugetropft. Man rührt nach beendeter Zugabe noch 4 Std. Die so erhaltene Emulsion wird am Vakuum auf das halbe Volumen eingeengt und danach mit 2 l Wasser verdünnt. Das ausgefallene produkt wird abgenutscht, mit Wasser gewaschen und am Vakuum getrocknet. Anschliessend löst man das Rohprodukt in 3 l heissem Acetonitril, filtriert die Lösung über HYFLO-Super-Cel® und engt die Lösung bei 60—70° am Wasserstrahlvakuum bis zur beginnenden Kristallisation ein. Man erhält so die Titelverbindung als bräunliche Kristalle mit einem Smp. von über 250°. IR-Spektrum (KBr-Pressling): 1355, 1695 und 1720 $cm^{-1}$.

Beispiel 10

a) 1-(4-Aminophenyl)-3-benzyl-3-azabicyclo[3.1.1]heptan-2,4-dion

Analog Beispiel la werden 2,46 g 3-Benzyl-1-(4-nitrophenyl)-3-azabicyclio[3.1.1]heptan-2,4-dion in 50 ml Essigester gelöst, in Gegenwart von 0,3 g 5%iger Palladium/Kohle hydriert und aufgearbeitet. Smp. 154—165,5° (aus Aether/Essigester). IR-Spektrum in $CHCl_3$: 1690 und 1745 $cm^{-1}$.

Herstellung des Ausgangsmaterials

b) 3-Benzyl-1-(4-nitrophenyl)-3-azabicyclo[3.1.1]heptan-2,4-dion

In 70 ml Dimethylformamid werden 4,92 g 1-(4-Nitrophenyl)-3-azabicyclo[3.1.1]heptan-2,4-dion gelöst und unter Stickstoffatmosphäre mit 1,2 g Natriumhydrid (prakt. Fluka) versetzt. Nach 30 Min. Rühren bei Raumtemperatur wird das Gemisch auf 0° gekühlt und tropfenweise mit einer Lösung von 4,5 g Benzylbromid in 10 ml Dimethylformamid versetzt. Nach beendeter Zugabe wird bei Raumptemperatur 4 Std. gerührt. Man zersetzt das überschüssige Natriumhydrid durch Zugabe von Methanol und dampft im Vakuum ein. Der Rückstand wird in Essigester aufgenommen und mit Wasser und wässriger Kochsalzlösung gewaschen. Nach dem Trocknen über Magnesiumsulfat wird abfiltriert und eingedampft. Smp. 150—152° (aus Aether). IR-Spektrum in $CHCl_3$: 1355, 1690 und 1745 $cm^{-1}$.

Beispiel 11

a) 1-(4-Aminophenyl)-3-cyclohexylmethyl-3-azabicyclo[3.1.1]heptan-2,4-dion

2,4 g Cis-1-(4-aminophenyl)-1,3-cyclobutan-dicarbonsäure und 1,3 ml Cyclohexylmethylamin werden in 100 ml Xylol bei 140° 24 Std. am Wasserabscheider gerührt. Das Reaktionsgemisch wird im Vakuum zur Trockene eingedampft und anschliessend mit Aether an Kieselgel chromatographiert. Die kristalline Fraktion ist mit der Titelverbindung von Beispiel 6a identisch.

Herstellung des Ausgangsmaterials

b) Cis-1-(4-aminophenyl)-1,3-cyclobutan-dicarbonsäure

5 g Cis-1-(4-nitrophenyl)-1,3-cyclobutandicarbonsäure werden in 170 ml 2-Methoxyäthanol gelöst und in Gegenwart von 0,5 g 5%iger Palladium/Kohle hydriert. Nach dem Filtrieren durch HYFLO-Super-Cel® wird eingedampft. Hellgelbe Kristalle, Smp. 228—229° (Zersetzung).

Beispiel 12

1-(4-Aminophenyl)-5-methyl-3-n-propyl-3-azabicyclo[3.1.1]heptan-2,4-dion

Eine Lösung von 10,0 g 1-(4-Nitrophenyl)-5-methyl-3-n-propyl-3-azabicyclo[3.1.1]-heptan-2,4-dion in 200 ml Essigester wird mit 1,0 g 5 %igem Palladium/Kohle-Katalysator versetzt und bei Normaldruck und 30—35° in Wasserstoffatmosphäre hydriert. Nach beendeter Wasserstoffaufnahme wird das Reaktionsgemisch it 100 ml Methylenchlorid verdünnt und durch Filtation über HYFLO-Super-Cel® vom Katalysator befreit. Man dampft im Vakuum das Lösungsmittel ab, kristallisiert den Rückstand auseinem Essigester/n-Hexan-Gemisch um und erhält so die Titelverbindung als weisse Kristalle mit einem Smp. von

135—136°. IR-Spektrum in $CHCl_3$: 1685 und 1740 $cm^{-1}$.

Herstellung des Ausgangsmaterials

b) 1-(4-Nitrophenyl)-5-methyl-3-n-propyl-3-azabicyclo[3.3.1]heptan-2,4-dion

Eine Lösung von 23,0 g 4-Aza-6-methyl-2-(4-nitrophenyl)-4-n-propyl-1,6-heptadien-3,5-dion und 0,23 g 2,6-Di-tert-butyl-p-kresol in 2,3 l Aceton wird während 3 Std. unter Rühren mit einer UV-Lampe (Philips 125 HPK) bestrahlt, welche in einem doppelwandigem, mit Wasser gekühltem, Pyrexglasschacht in die Reaktionslösung getaucht wird. Nach dem Eindampfen wird der Rückstand aus einem Methylenchlorid/Diisopropyläther-Gemisch umkristallisiert, und man erhält so die Titelverbindung als weisse Kristalle mit einem Smp. von 128,5—129,5°. IR-Spektrum in $CHCl_3$: 1350, 1685 und 1745 $cm^{-1}$.

c) 4-Aza-6-methyl-2-(4-nitrophenyl)-4-n-proyl-1,6-heptadien-3,5-dion

Zu einer gerührten Suspension von 23,2 g α-(4-Nitrophenyl)-acrylsäure in 1 ml Dimethylformamid und 600 ml Methylenchlorid wird während 30 Min. bei Raumptemperatur eine Lösung von 15,3 g Oxalylchlorid in 100 ml Methylenchlorid getropft. Es wird nach beendeter Zugabe noch 30 Min. gerührt, bis die Gasentwicklung beendet ist. Die so erhaltene Lösung von α-(4-Nitrophenyl)-acrylsäurechlorid wird auf 0° gekühlt und in eine auf 0—5° gekühlte Lösung von 15,2 g N-n-Propylmethacrylamid und 24,2 g Triäthylamin in 100 ml Methylenchlorid getropft. Nach beendeter Zugabe wird 1,5 Std. bei Raumtemperatur nachgerührt. Der am Vakuum eingedampfte Rückstand wird mit Aether verrührt, anschliessend filtriert und eingedampft.

Der Rückstand wird mit kochendem Hexan verrührt, mit Aktivkohle versetzt und heiss filtriert. Das Filtrat wird eingedampft und der Rückstand aus Diisopropyläther umkristallisiert. Die blassgelbe kristalline Titelverbindung hat einen Smp. von 62—63°.

Beispiel 13

a) 5-Aethyl-1-(4-aminophenyl)-3-n-propyl-3-azabicyclo[3.1.1]heptan-2,4-dion

Analog Beispiel 12a werden 6,3 g 5-Aethyl-1-(4-nitrophenyl)-3-n-propyl-3-azabicyclo[3.1.1]heptan-2,4-dion in 120 ml Essigester gelöst, in Gegenwart von 0,6 g 5%iger Palladium/Kohle hydriert und aufgearbeitet. Smp. 124,5—125° (aus Aether). IR-Spektrum in $CHCl_3$: 1685 und 1740 $cm^{-1}$.

Herstellung des Ausgangsmaterials

b) 5-Aethyl-1-(4-nitrophenyl)-3-n-propyl-3-azabicyclo[3.1.1]heptan-2,4-dion

Analog Beispiel 1b werden 29,6 g 6-Aetyl-4-aza-2-(4-nitrophenyl)-4-n-propyl-1,6-heptadien-3,5-dion und 0,27 g 2,6-Di-tert-butyl-p-kresol in 560 ml 1,3-Dichlorbenzol bei 170° gerührt und aufgearbeitet. Smp. 121—122° (aus Diisopropyläther). IR-Spektrum in $CHCl_3$: 1350, 1685 und 1740 $cm^{-1}$.

c) 6-Aethyl-4-aza-2-(4-nitrophenyl)-4-n-propyl-1,6-heptadien-3,5-dion

Die Titelverbindung wird ausgehend von 34,7 g α-(4-Nitrophenyl)-acrylsäure, 22,8 g Oxalylchlorid und 26 g N-n-Propyl-α-äthylacrylamid analog Beispiel 12c hergestellt4. Gelbes Oel, $R_f$(n-Hexan/Aether 1:1) = 0,46.

Beispiel 14

a) 1-(4-Aminophenyl)-5-isobutyl-3-n-propyl-3-azabicyclo[3.1.1]heptan-2,4-dion

Analog Beispiel 12a werden 5,2 g 5-Isobutyl-1-(4-nitrophenyl)-3-n-propyl-3-azabicyclo[3.1.1]heptan-2,4-dion in 100 ml Essigester in Gegenwart von 0,5 g 5%iger Palaldium/Kohle hydriert und aufgearbeitet. Smp. 86,5—87° (aus n-Hexan/Aether). IR-Spektrum in $CHCl_3$: 1690 und 1440 $cm^{-1}$.

Herstellung des Ausgangsmaterials

b) 5-Isobutyl-1-(4-nitrophenyl)-3-n-proyl-3-azabicyclo[3.1.1]heptan-2,4-dion

Analog Beispiel 12b werden 20,7 g 4-Aza-6-isobutyl-2-(4-nitrophenyl)-4-n-propyl-1,6-heptadien-3,5-dion in Gegenwart von 0,2 g 2,6-Di-tert-butyl-p-kresol in 1,5 l Aceton bestrahlt. Das eingedampfte Reaktionsgut wird an Kieselgel mit n-Hexan/Aether 1:1 chromatographiert. Smp. 87—88° (aus n-Hexan/Aether). IR-Spektrum in $CHCl_3$: 1355, 1690 und 1740 $cm^{-1}$.

c) 4-Aza-6-isobutyl-2-(4-nitrophenyl)-4-n-propyl-1,6-heptadien-3,5-dion

Die Titelverbindung wird ausgehend von 16,4 g α-(4-Nitrophenyl)-acrylsäure, 10,7 g Oxalylchlorid und 14,4 g N-n-propyl-α-isobutylacrylamid analog Beispiel 12c hergestellt; gelbes Oel.

Beispiel 15

a) 1-(4-Aminophenyl)-5-phenyl-3-n-propyl-3-azabicyclo[3.1.1]heptan-2,4-dion

Analog Beispiel 12a werden 1,8 g 1-(4-Nitrophenyl)-5-phenyl-3-n-propyl-3-azabicyclo[3.1.1]heptan-2,4-dion in 40 ml Essigester gelöst, in Gegenwart von 0,2 g 5%iger Palladium/Kohle hydriert und aufgearbeitet. Smp. 144—145° (aus n-Hexan/Aether). IR-Spektrum in $CHCl_3$: 1685 und 1735 $cm^{-1}$.

Herstellung des Ausgangsmaterials

b) 1-(4-Nitrophenyl)-5-phenyl-3-n-propyl-3-azabicyclo[3.1.1]heptan-2,4-dion

Analog Beispiel 12b werden 3,6 g 4-Aza-2-(4-nitrophenyl)-6-phenyl-4-n-propyl-1,6-heptadien-3,5-dion in Gegenwart von 0,04 g 2,6-Di-tert-butyl-p-kresol in 250 ml Aceton bestrahlt. Das eingedampfte Reaktionsgut wird an Kieselgel mit Toluol/Essigester 15:1 chromatographiert. Smp. 144—146°C (aus Toluol/aether). IR-Spektrum in $CHCl_3$: 1350, 1695 und 1745 $cm^{-1}$.

c) 4-Aza-2-(4-nitrophenyl)-6-phenyl-4-n-propyl-1,6-heptadien-3,5-dion

Die Titelverbindung wird ausgehend von 36,7 g α-(4-Nitrophenyl)acrylsäure, 24,1 g Oxyalylchlorid und 35,8 g N-n-propyl-α-phenylacrylamid analog Beispiel 12c hergstelt; weisse Kristalle mit einem Smp. von 98—99°.

Beispiel 16

a) 1,5-Di-(4-aminophenyl)-3-n-propyl-3-azabicyclo[3.1.1]heptan-2,4-dion

Analog Beispiel 12a werden 0,4 g 1,5-Di-(4-nitrophenyl)-3-n-propyl-azabicyclo[3.1.1]heptan-2,4-dion in 15 ml Essigester gelöst, in Gegenwart von 80 mg 5%iger Palladium/Kohle hydriert und aufgearbeitet. Smp. 149—150° (aus Aether/Methylenchlorid). IR-Spektrum in $CHCl_3$: 1685 und 1735 $cm^{-1}$.

Herstellung des Ausgangsmaterials

b) 1,5-Di-(4-nitrophenyl)-3-n-propyl-azabicyclo[3.1.1]heptan-2,4-dion

Analog Beispiel 1b werden 6 g 4-Aza-2,6-di(4-nitrophenyl)-4-n-propyl-1,6-heptadien-3,5-dion und 0,08 g 2,6-Di-tert-butyl-p-kresol in 85 ml 1,3-Dichlorbenzol bei 170° gerührt und aufgearbeitet. Aus einer Säulenchromatographie des Rohproduktes an Kieselgel mit Toluol/essigester 4:1 erhält man die Titelverbindung, welche nach Umkristallisation aus einem Toluol/Aether-Gemisch bei 237—238° schmilzt. IR-Spektrum in $CHCl_3$: 1350, 1690 und 1740 $cm^{-1}$.

c) 4-Aza-2,6-di-(4-nitrophenyl)-4-n-propyl-1,6-heptadien-3,5-dion

Die Titelverbindung wird ausgehend von 19,3 g α-(4-Nitrophenyl)-acrylsäure, 12,7 g Oxalylchlorid und 18 g N-n-Propyl-α-(4-nitrophenyl)-acrylamid analog Beispiel 12c hergestellt. Weisse Kristalle mit einem Smp. von 177—178°.

Beispiel 17

a) 1-(4-Aminophenyl)-5-methyl-3-azabicyclo[3.1.1]heptan-2,4-dion

Analog Beispiel 12a werden 3,7 g 5-Methyl-1-(4-nitrophenyl)-3-azabicyclo[3.1.1]heptan-2,4-dion in 80 ml 2-Methoxyäthanol in Gegenwart von 0,4 g 5%iger Palladium/Kohle hydriert und aufgearbeitet. Smp. 216° (aus Aethanol). IR-Spektrum in $CHCl_3$: 1715 und 1745 $cm^{-1}$.

Herstellung des Ausgangsmaterials

b) 5-Methyl-1-(4-nitrophenyl)-3-azabicyclo[3.1.1]heptan-2,4-dion

In die gerührte Suspension von 0,9 g 3-Methyl-1-(4-nitrophenyl)-1,3-cyclobutandicarbonsäureanhydrid in 50 ml 1,3-Dichlorbenzol wird bei 60° Ammoniak eingeleitet. Nach dem Ausfallen der Amidosäure wird 8 Std. am Rückfluss erwärmt und die Reaktionslösung anschliessend heiss abgenutscht. Beim Abkühlen fällt die Titelverbindung in Form braungelber Kristalle aus. Smp. 242—244° (aus Essigester). IR-Spektrum (KBr-Pressling): 1360, 1700 und 1745 $cm^{-1}$.

c) 3-Methyl-1-(4-nitrophenyl)-1,3-cyclobutan-dicarbonsäureanhydrid

Ein Gemisch von 10 g cis-3-Methyl-1-(4-nitrophenyl)-1,3-cyclobutandicarbonsäure und 100 ml Essigsäureanhydrid wird während 20 Std. unter Rückfluss erwärmt. Das Reaktionsgemisch wird zur Trockene eingedampft, der Rückstand in Toluol suspendiert, abgenutscht, mit Aether gewaschen und am Vakuum getrocknet. Man erhält die Titelverbindung als graue Kristalle mit einem Smp. von 201—202°. IR-Spektrum (KBr-Pressling): 1350, 1765 und 1820 $cm^{-1}$.

d) cis-3-Methyl-1-(4-nitrophenyl)-1,3-cyclobutandicarbonsäure

Eine Suspension von 46 g 5-Methyl-1-(4-nitrophenyl)-3-n-propyl-3-azabicyclo[3.1.1]heptan-2,4-dion, 250 ml Essigsäure und 500 ml 50%iger Schwefelsäure wird während 20 Std. bei 140° gerührt. Nach dem Abkühlen wird auf Eis gegossen und dreimal mit Aether extrahiert. Die Aetherauszüge werden mit Wasser gewaschen, mit Magnesiumsulfat getrocknet und eingedampft. Die Titelverbindung erhält man durch Umkristillisation aus Essigester, Smp. 231° (Zersetzung).

Beispiel 18

1-(4-Acetylaminophenyl)-3-n-propyl-3-azabicyclo[3.1.1]heptan-2,4-dion

Eine Lösung von 260 mg 1-(4-Aminophenyl)-3-n-propyl-3-azabicyclo[3.1.1]heptan-2,4-dion und 6 mg 4-Dimethylaminopyridin in 8 ml Tetrahydrofuran wird tropfenweise mit einer Lösung von 0,11 ml Acetanhydrid in 0,5 ml Tetrahydrofuran versetzt. Nach $2\frac{1}{2}$ Std. Nachrühren bei Raumtemperatur wird as Reaktionsgemisch mit 2 Tropfen Aethanol versetzt und weitere 15 Min. gerührt. Nach dem Eindampfen und anschliessender Umkristallisation aus Essigester/Petroläther erhält man die Titelverbindung als weisse Kristalle mit Smp. 138,5—139,5°, $R_f$(Methylenchlorid/Methanol/Eisessig 40:5:1) = 0,55.

Beispiel 19

1-(4-Dimethylaminophenyl)- und 1-(4-Methylaminophenyl)-3-n-propyl-3-azabicyclo[3.1.1]heptan-2,4-dion

Eine Lösung von 20,3 g 1-(4-Aminophenyl)-3-n-propyl-3-azabicyclo[3.1.1]heptan-2,4-dion in 470 ml Tetrahydrofuran versetzt man unter Rühren mit einer Lösung von 15 ml Dimethylsulfat in 60 ml Tetrahydrofuran. Nun wird unter Rühren innerhalb 6 Std. eine Lösung von 21,9 ml Triaethylamin in 40 ml Tetrahydrofuran zugetropft. Nach 10 Std. Nachrührzeit wird das Reaktionsgemisch mit 2,5 ml 15% wässriger Ammoniak-Lösung versetzt. Nach dem Eindampfen wird der Rückstand mit Wasser versetzt und mit Methylenchlorid extrahiert. Die organische Phase wird abgetrennt, über Magnesiumsulfat getrocknet, filtriert und eingedampft. Nach einer Chromatographie über Kieselgel mit Hexan/Essigester 2:1 erhält man 1-(4-Dimethylaminophenyl)-3-n-propyl-3-azabicyclo[3.1.1]heptan-2,4-dion, $R_f$(Hexan/Essigester 1:1) = 0,45, Smp. 139—140°C (aus Aethanol), sowie 1-(4-Methylaminophenyl)-3-n-propyl-3-azabicylo[3.1.1]heptan-2,4-dion, $R_f$(Hexan/Essigester 1:1) = 0,35, Smp. 134—135°C (aus Aethanol).

Beispiel 20

1-(4-N-Acetyl-N-methyl-aminophenyl)-3-n-propyl-3-azabicyclo[3.1.1]heptan-2,4-dion

Ein Gemisch von 272 mg 1-(4-N-Methylaminophenyl)-3-n-propyl-3-azabicyclo[3.1.1]heptan-2,4-dion, 10 ml Tetrahydrofuran, 6 mg 4-Dimethylaminopyridin und 0,11 ml Acetanhydrid wird 2 Std. bei Raumtemperatur gerührt. Dann wird das Reaktionsgemisch mit 2 Tropfen Methanol versetzt, weitere 15 Min. gerührt und anschliessend eingedampft. Der Rückstand wird zweimal zwischen Essigester und Wasser verteilt. Die vereinigten, über $MgSO_4$ getrockneten organischen Phasen werden filtriert und eingedampft. Der ölige Rückstand wird aus Essigester/Petroläther kristallisiert. Man erhält so die Titelverbindung als weisse Kristalle von Smp. 144—146°, $R_f$(Methylenchlorid/Methanol 10:1) = 0,55.

Beispiel 21

1-(4-Methansulphonylaminophenyl)-3-n-propyl-3-azabicyclo[3.1.1]heptan-2,4-dion

Eine Lösung von 1,03 g 1-(4-Aminophenyl)-3-n-propyl-3-azabicyclo[3.1.1]heptan-2,4-dion und 24 mg 4-Dimethylaminopyridin in 10 ml Pyridin wird bei Raumtemperatur mit einer Lösung von 0,31 ml Methansulfonsäurechlorid in 3 ml Methylenchlorid versetzt. Nach 5 Std. Nachrührzeit wird mit 50 ml Wasser versetzt und über Nacht bei 0—5°C stehen gelassen. Man extrahiert mit Methylenchlorid, wäscht die organische Phase hintereinander mit Wasser, 2N kalter Salzsäure, Wasser, halbgesättigter Bicarbonatlösung und Wasser. Nach dem Trocknen über Magnesiumsulfat wird filtriert, eingedampft und der Rückstand aus Methanol umkristallisiert. Man erhält so die Titelverbindung als weisse Kristalle mit Smp. 159—160°C, $R_f$(Hexan/Essigester 1:1) = 0,15.

Beispiel 22

a) 3-Aethyl-1-(4-aminophenyl)-3-azabicyclo[3.1.1]heptan-2,4-dion

Analog Beispiel Ia werden 2,33 g 3-Aethyl-1-(4-nitrophenyl)-3-azabicyclo[3.1.1]heptan-2,4-dion in 70 ml Aethanol in Gegenwart von 0,15 g Palladium/Kohle hydriert und aufgearbeitet. Smp. 159—162° (aus Essigester/Petroläther).

Herstellung des Ausgangsmaterials

b) 3-Aethyl-1-(4-nitrophenyl)-3-azabicyclo[3.1.1]heptan-2,4-dion

Eine Lösung von 2,46 g 1-(4-Nitrophenyl)-3-azabicyclo[3.1.1]heptan-2,4-dion in 25 ml N,N-Dimethylformamid wird mit 0,36 g Natriumhydrid versetzt und 30 Min. bei Raumtemperatur gerührt. Anschliessend werden 2,33 g Aethyljodid gelöst in 10 ml N,N-Dimethylformamid zugetropft. Nach beendeter Reaktion wird das Reaktionsgemisch vom N,N-Dimethylformamid befreit. Den Rückstand verteilt man zwischen Essigester und Wasser, trocknet die organische Phase über Magnesiumsulfat und erhält so nach dem Eindampfen das Produkt als Feststoff, Smp. 175—179°, $R_f$(Essigester/Hexan 4:1) = 0,52.

Beispiel 23

a) 1-(4-Aminophenyl)-3-n-butyl-3-azabicyclo[3.1.1]heptan-2,4-dion

Analog Beispiel 1 werden 2,08 g 3-n-Butyl-1-(4-nitrophenyl)-3-azabicyclo[3.1.1]heptan-2,4-dion in 60 ml Aethanol in Gegenwart von 0,15 g Palladium/Kohle hydriert und aufgearbeitet. Smp. 178—179° (aus Essigester).

Herstellung des Ausgangsmaterials

b) 3-n-Butyl-1-(4-nitrophenyl)-3-azabicyclo[3.1.1]heptan-2,4-dion

Eine Lösung von 2,46 g 1-(4-Nitrophenyl)-3-azabicyclo[3.1.1]heptan-2,4-dion in 25 ml N,N-Dimethylformamid wird mit 0,36 g Natriumhydrid versetzt und 30 Min. bei Raumtemperatur gerührt. Anschliessend werden 1,6 ml n-Butylbromid gelöst in 10 ml, N,N-Dimethylformamid zugetropft. Nach beendeter Reaktion wird das Reaktionsgemisch vom N,N-Dimethylformamid befreit. Den Rückstand verteilt man zwischen Essigester und Wasser, trocknet die organische Phase über Magnesiumsulfat und erhält so nach dem Eindampfen und anschliessender Umkristallisation aus Essigester/Petrolaether das Produkt von Smp. 120—123°.

Beispiel 24

a) 1-(4-Aminophenyl)-3-isobutyl-3-azabicyclo[3.1.1]heptan-2,4-dion

Analog Beispiel 1 werden 2,0 g 3-Isobutyl-1-(4-nitrophenyl)-3-azabicyclo[3.1.1]heptan-2,4-dion in 100 ml Aethanol in Gegenwart von 0,1 g Palladium/Kohle hydriert und aufgearbeitet. Smp. 158—160° (aus Essigester/Petroläther).

Herstellung des Ausgangsmaterials

b) 3-Isobutyl-1-(4-nitrophenyl)-3-azabicyclo[3.1.1]heptan-2,4-dion

Eine Lösung von 2,46 g 1-(4-Nitrophenyl)-3-azabicyclo[3.1.1]heptan-2,4-dion in 25 ml N,N-Dimethylformamid wird mit 0,36 g Natriumhydrid versetzt und 30 Min. bei Raumtemperatur gerührt. Anschliessend werden 2,76 g Isobutyljodid gelöst in 10 ml N,N-Dimethylformamid zugetropft. Nach beendeter Reaktion wird das Reaktionsgemisch vom N,N-Dimethylformamid befreit. Den Rückstand verteilt man zwische Essigester und Wasser, trocknet die organische Phase über Magnesiumsulfat und erhält so nach dem Eindampfen und chromatographischer Reinigung auf Kieselgel im Systemn Essigester/Hexan 1:2 das Produkt als Feststoff, Smp. 136—137°C, $R_f$(Essigester/Hexan 4:1) = 0,6.

Beispiel 25

a) 1-(4-Aminophenyl)-3-n-pentyl-3-azabicyclo[3.1.1]heptan-2,4-dion

Analog Beispiel 1 werden 2,58 g 1-(4-Nitrophenyl)-3-n-pentyl-3-azabicyclo[3.1.1]heptan-2,4-dion in 75 ml Aethanol in Gegenwart von 0,15 g Palladium/Kohle hydriert und aufgearbeitet. Smp. 92—94° (aus Essigester/Petroläther).

Herstellung des Ausgangsmaterials

b) 1-(4-Nitrophenyl)-3-n-phenyl-3-azabicyclo[3.1.1]heptan-2,4-dion

Eine Lösung von 2,46 g 1-(4-Nitrophenyl)-3-azabicyclo[3.1.1]heptan-2,4-dion in 25 ml N,N-Dimethylformamid wird mit 0,36 g Natriumhydrid versetzt und 30 Min. bei Raumtemperatur gerührt. Anschliessend werden 2,96 g n-Pentyljodid gelöst in 10 ml N,N-Dimethylformamid zugetropft. Nach beendeter Reaktion wird das Reaktionsgemisch vom N,N-Dimethylformamid befreit. Den Rückstand verteil man zwischen Essigester und Wasser, trocknet die organische Phase über Magnesiumsulfat und erhält so nach dem Eindampfen das Produkt vom Smp. 75—79°.

Beispiel 26

a) 1-(4-Aminophenyl)-3-n-heptyl-3-azabicyclo[3.1.1]heptan-2,4-dion

Analog Beispiel 1 werden 2,1 g 3-n-Heptyl-1-(4-nitrophenyl)-3-azabicyclo[3.1.1]heptan-2,4-dion in 100 ml Aethanol in Gegenwart von 0,1 g Palladium/Kohle hydriert und aufgearbeitet. Man erhält so die Titelverbindung als wachsartige Kristalle nach säulenchromatographischer Reinigung auf Kieselgel mit dem System Hexan/Essigester 1:1, Smp. 69—71°, $R_f$(Hexan/Essigester 1:1) = 0,25.

Herstellung des Ausgangsmaterials

b) 3-n-Heptyl-1-(4-nitrophenyl)-3-azabicyclo[3.1.1]heptan-2,4-dion

Eine Lösung von 2,46 g 1-(4-Nitrophenyl)-3-azabicyclo[3.1.1]heptan-2,4-dion in 25 ml N,N-Dimethylformamid wird mit 0,36 g Natriumhydrid versetzt und 30 Min. bei Raumtemperatur gerührt. Anschliessent werden 2,68 g n-Heptylbromid gelöst in 10 ml N,N-Dimethylformamid zugetropft. Nach beendeter Reaktion wird das Reaktionsgemisch vom N,N-Dimethylformamid befreit. Den Rückstand verteilt man zwischen Essigester und Wasser, trocknet die organische Phase über Magnesiumsulfat und erhält, so nach dem Eindampfen und chromatographischer Reinigung auf Kieselgel im System Essigester/Hexan 2:5 das Produkt als Feststoff, Smp. 91—92°, $R_f$(Essigester/Hexan 4:1) = 0,63.

Beispiel 27

a) 3-Allyl-(4-aminophenyl)-3-azabicyclo[3.1.1]heptan-2,4-dion

Ein Gemisch von 1,97 g 3-Allyl-1-(4-nitrophenyl)-3-azabicyclo[3.1.1]heptan-2,4-dion und 5,2 g Zinnpulver in 14 ml Wasser und 14 ml konzentrierter Salzsäure wird 1,5 Std. bei 100° gerührt. Nach Abkühlung auf Raumtemperatur wird das Reaktionsgemisch mit etwas Wasser verdünnt, filtriert und durch Zugabe von Natronlauge alkalisch gestellt. Man extrahiert das Reaktionsgemisch mit Essigester, wäscht die organische Phase mit verdünnter Kochsalzlösung neutral, trocknet über Magnesiumsulfat und dampft ein. Smp. 176—178° (aus Essigester/Petrolaether).

Herstellung des Ausgangsmaterials

b) 3-Allyl-1-(4-nitrophenyl)-3-azabicyclo[3.1.1]heptan-2,4-dion

Eine Lösung von 2,46 g 1-(4-Nitrophenyl)-3-azabicyclo[3.1.1]heptan-2,4-dion in 25 ml N,N-Dimethylformamid wird mit 0,36 g Natriumhydrid versetzt und 30 Min. bei Raumtemperatur gerührt. Anschliessend werden 1,27 ml Allylbromid gelöst in 10 ml N,N-Dimethylformamid zugetropft. Nach beendeter Reaktion wird das Reaktionsgemisch vom N,N-Dimethylformamid befreit. Den Rückstand verteilt man zwischen Essigester und Wasser, trocknet die organische Phase über Magnesiumsulfat und

erhält so nach dem Eindampfen und anschliessender Umkristallisation aus Essigester/Petroläther das Produkt vom Smp. 146—147°.

Beispiel 28

a) 1-(4-Aminophenyl)-3-propargyl-3-azabicyclo[3.1.1]heptan-2,4-dion

Ein Gemisch von 1,6 g 1-(4-Nitrophenyl)-3-propargyl-3-azabicyclo[3.1.1]heptan-2,4-dion und 4,2 g Zinnpulver in 12 ml Wasser und 12 ml konzentrierter Salzsäure wird eine Stunden bei 100° gerührt. Nach Abkühlung auf Raumtemperatur wird das Reatkionsgemisch mit etwas Wasser verdünnt, filtriert und durch Zugabe von Natronlauge alkalisch gestellt. Man extrahiert das Reaktionsgemisch mit Essigester, wäscht die organische Phase mit verdünnter Kochsalzlösung neutral, trocknet über Magnesiumsulfat und dampft ein. Nach chromatographischer Reinigung an Kieselgel im System Essigester/Hexan 1:1 erhält man die Titelverbindung, $R_f$(Essigester/Hexan 1:1) = 0,15.

Herstellung des Ausgangsmaterials

b) 1-(4-Nitrophenyl)-3-propargyl-3-azabicyclo[3.1.1]heptan-2,4-dion

Eine Lösung von 2,46 g 1-(4-Nitrophenyl)-3-azabicyclo[3.1.1]heptan-2,4-dion in 25 ml N,N-Dimethylformamid wird mit 0,36 g Natriumhydrid versetzt und 30 Min. bei Raumtemperatur gerührt. Anschliessend werden 0,97 ml Propargylbromid gelöst in 10 ml N,N-Dimethylformamid zugetropft. Nach beendeter Reaktion wird das Reaktionsgemisch vom N,N-Dimethylformamid befreit. Den Rückstand verteilt man zwischen Essigester und Wasser, trocknet die organische Phase über Magnesiumsulfat und erhält so nach dem Eindampfen und anschliessender Umkristallisation aus Essigester/Petroläther das Produkt vom Smp. 166—168°.

Beispiel 29

Lacktabletten, enthaltend 300 mg 1-(4-Aminophenyl)-3-n-propyl-3-azabicyclo[3.1.1]heptan-2,4-dion, können wie folgt hergestellt werden:

Zusammensetzung für 10 000 Tabletten

| | |
|---|---|
| 1-(4-Aminophenyl)-3-n-propyl-3-azabicyclo[3.1.1]heptan-2,4-dion | 3000,0 g |
| Maisstärke | 680,0 g |
| Kolloidale Kieselsäure | 200,0 g |
| Magnesiumstearat | 20,0 g |
| Stearinsäure | 50,0 g |
| Natriumcarboxymethylstärke | 250,0 g |
| Wasser | q.s |

Ein Gemisch von 1-(4-Aminophenyl)-3-n-propyl-3-azabicyclo[3.1.1]heptan-2,4-dion, 50 g Maisstärke und der kollodialen Kieselsäure wird mit Stärkekleister aus 250 g Miasstärke und 2,2 kg entmineralisiertem Wasser zu einer feuchten Masser verarbeitet. Diese wird durch ein Sieb von 3 mm Maschenweite getrieben und bei 45° während 30 Minuten im Wirbelschichttrockner getrocknet. Das trockene Granulat wird durch ein Sieb von 1 mm Maschenweite gedrückt, mit einer vorher gesiebten Mischung (1 mm Sieb) von 330 g Maisstärke, des Magnesiumstearats, der Stearinsäure und der Natriumcarboxymethylstärke gemischt und zu schwach gewölbten Tabletten verpresst.

Die Presslinge werden in einem Dragierkessel von 45 cm Durchmesser mit einer ösung 20 g Schellack und 40 g Hydroxypropylmethylcellulose (niedere Viskosität) in 110 g Methanol und 1350 g Methylenchlorid durch gleichmässiges Aufsprühen während 30 Minuten überzogen; dabei wird durch gleichzeitiges Einblasen von Luft von 60° getrocknet.

Anstelle des oben genannten Wirkstoffes kann man auch dieselbe Menge eines anderen erfindungsgemässen Wirkstoffes verwenden.

**Patentansprüche für die Vertragsstaaten: BE CH DE FR GB IT LI LU NL SE**

1. Verbindungen der Formel

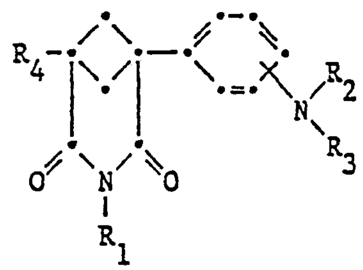

(I),

worin $R_1$ Wasserstoff, Alkyl mit 1—12 C-Atomen, Alkenyl mit 2—12 C-Atomen, Niederalkinyl, Cycloalkyl oder Cycloalkenyl mit 3—10 C-Atomen, Cycloalkylniederalkyl mit 4—10 C-Atomen, Cycloalkylniederalkenyl mit 5—10 C-Atomen, Cycloalkenylniederalkyl mit 4—10 C-Atomen oder unsubstituiertes oder durch Niederalkyl, Hydroxy, Niederalkoxy, Acyloxy, Amino, Niederalkylamino, Diniederalkylamino, Acylamino oder Halogen substituiertes Aryl mit 6—12 C-Atomen oder Arylniederalkyl mit 7—15 Atomen, $R_2$ Wasserstoff, Niederalkyl, Sulfo, Niederalkanoyl oder Niederalkansulfonyl, $R_3$ Wasserstoff oder Niederalkyl und $R_4$ Wasserstoff, Niederalkyl, Phenyl oder durch —$N(R_2)(R_3)$ substituiertes Phenyl bedeuten, sowie Salze davon, wobei die mit "nieder" bezeichneten Reste bis einschliesslich 7 Kohlenstoffatome enthalten.

2. Verbindungen gemäss Anspruch 1 der Formel I, worin $R_1$ Wasserstoff, Alkyl bis einschliesslich 12 C-Atome, Niederalkenyl, Niederalkinyl, Cycloalkyl mit 3—6 C-Atomen, unsubstituiertes Cycloalkylniederalkylmit 4—7 C-Atomen, oder durch Niederalkyl, Hydroxy, Niederalkoxy, Acyloxy, Amino, Niederalkylamino, Diniederalkylamino, Acylamino oder Halogen substituiertes Arylniederalkyl mit 7 oder 8 C-Atomen, $R_2$ Wasserstoff, Niederalkyl, Niederalkanoyl oder Niederalkansulfonyl, $R_3$ Wasserstoff oder Niederalkyl und $R_4$ Wasserstoff, Niederalkyl, Phenyl oder durch —$N(R_2)(R_3)$ substituiertes Phenyl bedeuten, sowie Salze davon, wobei die mit "nieder" bezeichneten Reste bis einschliesslich 7 Kohlenstoffatome enthalten.

3. Verbindungen gemäss Anspruch 1 der Formel I, worin $R_1$ Wasserstoff, Niederalkyl, Niederalkenyl, Niederalkinyl, Cycloalkyl mit 3—6 C-Atomen, oder Cycloalkylniederalkyl mit 4—7 C-Atomen, $R_2$ und $R_3$ Wasserstoff und $R_4$ Wasserstoff, Niederalkyl, Phenyl oder durch —$N(R_2)(R_3)$ substituiertes Phenyl bedeuten, sowie pharmazeutisch verwendbare Salze davon, wobei die mit "nieder" bezeichneten Reste bis einschliesslich 7 Kohlenstoffatome enthalten.

4. Verbindungen gemäss Anspruch 1 der Formel I, worin die Gruppe —$N(R_2)(R_3)$ sich in 4-Stellung des Phenylrestes befindet, $R_1$ Wasserstoff, Niederalkyl, Niederalkenyl, Niederalkinyl oder Cycloalkylniederalkyl mit 4—7 C-Atomen, $R_2$ und $R_3$ Wasserstoff und $R_4$ Wasserstoff oder Niederalkyl bedeuten, sowei pharmazeutisch verwendbare Salze davon, wobei die mit "nieder" bezeichneten Reste bis einschliesslich 7 Kohlenstoffatome enthalten.

5. Verbindungen gemäss Anspruch 1 der Formel I, worin die Gruppe —$N(R_2)(R_3)$ sich in 4-Stellung des Phenylrestes befindet, $R_1$ Wasserstoff, Alkyl bis einschliesslich 12 C-Atome, Niederalkenyl, Niederalkinyl, Cycloalkyl mit 3—6 C-Atomen, Cycloalkylmethyl mit 4—7 C-Atomen, oder unsubstituiertes oder substituiertes Benzyl und $R_2$, $R_3$ und $R_4$ Wasserstoff bedeuten, sowie pharmazeutisch verwendbare Salze davon, wobei die mit "nieder" bezeichneten Reste bis einschliesslich 7 Kohlenstoffatome enthalten.

6. 1-(4-Aminophenyl)-3-n-propyl-3-azabicyclo[3.1.1]heptan-2,4-dion gemäss Anspruch 1.

7. 1-(4-Aminophenyl)-3-methyl-3-azabicyclo[3.1.1]heptan-2,4-dion gemäss Anspruch 1.

8. 1-(4-Aminophenyl)-3-n-decyl-3-azabicyclo[3.1.1]heptan-2,4-dion gemäss Anspruch 1.

9. 1-(4-Aminophenyl)-3-cyclohexyl-3-azabicyclo[3.1.1]heptan-2,4-dion gemäss Anspruch 1.

10. 1-(4-Aminophenyl)-3-cyclohexylmethyl-3-azabicyclo[3.1.1]heptan-2,4-dion gemäss Anspruch 1.

11. Pharmazeutische Präparate enthaltend Verbindungen der Formel I gemäss Anspruch 1 oder Salze davon zusammen mit einem Trägerstoff.

12. Verbindungen der Formel I gemäss Anspruch 1 oder Salze davon zur Anwendung in einem Verfahren zur therapeutischen Behandlung des menschlichen oder tierischen Körpers.

13. Verbindungen der Formel I gemäss Anspruch 1 oder Salze davon zur Anwendung als carcinostatische Heilmittel.

14. Verfahren zur Herstellung von Verbindungen der Formel I und Salzen davon gemäss Anspruch 1, dadurch gekennzeichnet, dass man

a) in einer Verbindung der Formel

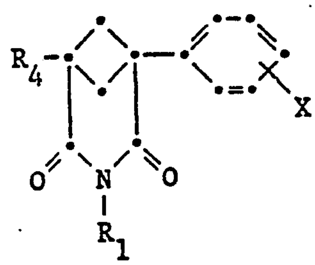

(II),

worin $R_1$ und $R_4$ die unter Formel I genannte Bedeutung haben und X eine Stickstoff enthaltende, reduzierbare Gruppe, Halogen, Carbamoyl, Azidocarbonyl oder eine geschützte Aminogruppe ist, oder in einem Salz davon X in die

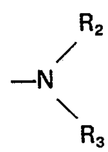

Gruppe überführt, oder

b) eine Verbindung der Formel

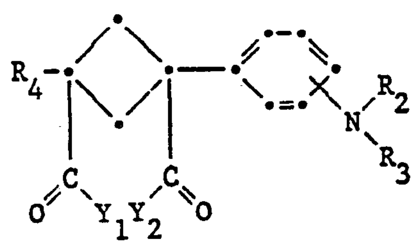

(III),

worin $R_2$, $R_3$ und $R_4$ die unter Formel I genannten Bedeutungen haben, $Y_1$ und $R_2$ unabhängig voneinander Hydroxy, Halogen, Niederalkoxy, Silyloxy oder Sulfonyloxy oder zusammen Sauerstoff darstellen, oder ein Salz davon durch Umsetzung mit einer die Gruppe $>N—R_1$ liefernde Verbindung zyklisiert oder

c) eine Verbindung der Formel

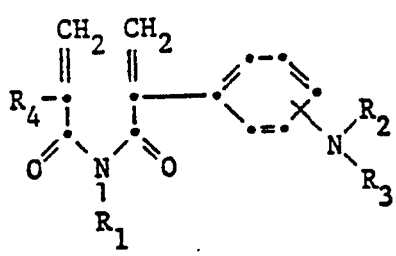

(IV),

worin $R_1$, $R_2$, $R_3$ und $R_4$ die unter Formel I genannte Bedeutung haben, oder ein Salz davon thermisch in Abwesenheit bzw. Anwesenheit eines inerten Lösungsmittels oder auch photochemisch zusammen mit

einem Triplett-Sensibilisatoren in einem photochemisch inerten Lösungsmittel zyklisiert, oder

d) eine erbindung der Formel

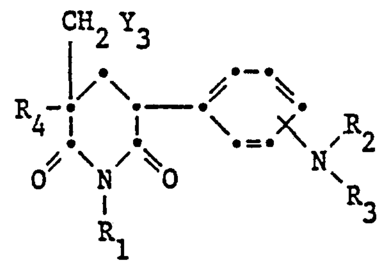

oder

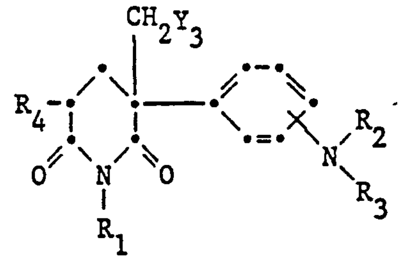

(V) (VI)

worin $R_1$, $R_2$, $R_3$ und $R_4$ die unter Formel I genannte Bedeutung haben und $Y_3$ Halogen oder Sulfonyloxy bedeutet, in Gegenwart einer nichtnuckleophilen Base zyklisiert und gewünschtenfalls ein erhältliches Salz in die freie Verbindung oder in ein anderes Salz überführt, und/oder eine erhältliche freie Verbindung in ein Salz überführt und/oder ein erhältliches Gemisch von Isomeren in die einzelnen Isomere auftrennt.

15. Verbindungen der Formel

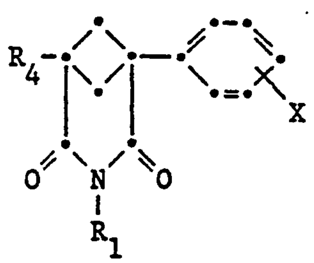

(II),

worin $R_1$ und $R_4$ die unter Formel I in Anspruch 1 genannte Bedeutung haben und X eine im Anspruch 14 definierte Gruppe darstellt, und Salze von diesen Verbindungen.

16. Verbindungen gemäss Anspruch 15 der Formel II, worin $R_1$ und $R_4$ die unter Formel I in Anspruch 1 genannte Bedeutung haben und X eine Nitrogruppe darstellt.

17. Verfahren zur Herstellung von Verbindungen der Formel II und Salzen davon gemäss Anspruch 15, dadurch gekennzeichnet, dass man

e) eine Verbindung der Formel

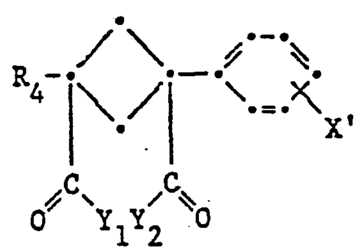

(VII),

worin $R_4$ die unter Formel I im Anspruch 1 genannte Bedeutung hat und $Y_1$ und $Y_2$ unabhängig voneinander Hydroxy, Halogen, Niederalkoxy, Silyloxy oder Sulfonyloxy oder zusammen Sauerstoff und X' eine der unter Formel II in Anspruch 14 angegebenen Gruppen X oder Carboxyl darstellen, oder ein Salz davon durch Umsetzung mit einer die Gruppe

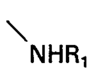

liefernden Verbindung zyklisiert, oder

f) eine Verbindung der Formel

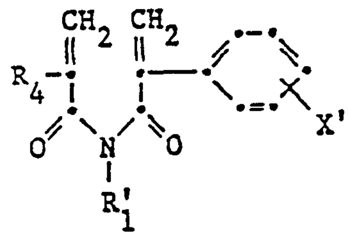 (VIII),

worin $R_1'$ eine Gruppe $R_1$ oder eine Aminoschutzgruppe und X′ eine Gruppe X oder Carboxyl darstellen und $R_1$, $R_4$ und X die oben genannten Bedeutungen haben, oder ein Salz davon thermisch in Abwesenheit bzw. Anwesenheit eines inerten Lösungsmittel oder auch photochemisch zusammen mit einem Triplett-Sensibilisatoren in einem photochemisch inerten Lösungsmittel zyklisiert

g) eine Verbindung der Formel

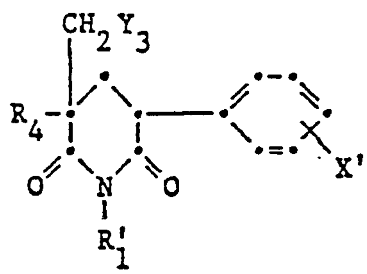 oder 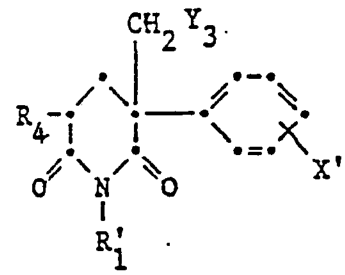

(IX) . (X)

worin $R_1'$ eine Gruppe $R_1$ oder eine Aminoschutzgruppe, X′ eine Gruppe X oder Carboxyl und $Y_3$ Halogen oder Sulfonyloxy bedeutet, in Gegenwart einer Nicht-nukleophilen Base zyklisiert und in einer erhältlichen Verbindung die Carboxylgruppe X′ in Carbamoyl oder Azidocarbonyl überführt und/oder die Aminoschutzgruppe $R_1'$ abspaltet und gewünschtenfalls ein erhältliches Salz in die freie Verbindung oder in ein anderes Salz überführt und/oder eine erhältliche freie Verbindung in ein Salz überführt und/oder ein erhältliches Gemisch von Isomeren in die einzelnen Isomere auftrennt.

18. Verwendung von Verbindungen der Formel I gemäss Anspruch 1 zur Herstellung eines Arzneimittels für die Behandlung hormonabhängiger Tumoren und hormonaler Anomalien.

**Patentansprüche für den Vertragsstaat: AT**

1. Verfahren zur Hestellung von Verbindungen der Formel

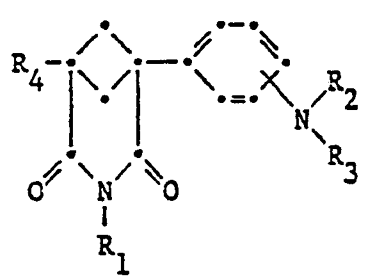 (I),

worin $R_1$ Wasserstoff, Alkyl mit 1—12 C-Atomen, Alkenyl mit 2—12 C-Atomen, Niederalkinyl, Cycloalkyl oder Cycloalkenyl mit 3—10 C-Atomen, Cycloalkylniederalkyl mit 4—10 C-Atomen, Cycloalkylniederalkenyl mit 5—10 C-Atomen, Cycloalkenylniederalkyl mit 4—10 C-Atomen oder unsubstituiertes oder durch Niederalkyl, Hydroxy, Niederalkoxy, Acyloxy, Amino, Niederalkylamino, Diniederalkylamino, Acylamino oder Halogen substituiertes Aryl mit 6—12 C-Atomen oder Arylniederalkylmit 7—15 Atomen, $R_2$ Wasserstoff, Niederalkyl, Sulfo, Niederalkanoyl oder Niederalkansulfonyl, $R_3$ Wasserstoff oder Niederalkyl und $R_4$ Wasserstoff, Niederalkyl, Phenyl oder durch $—N(R_2)(R_3)$ substituiertes Phenyl bedeuten, sowie Salze

davon, wobei die mit "nieder" bezeichneten Reste bis einschliesslich 7 Kohlenstoffatome enthalten, dadurch gekennzeichnet, dass man

a) in einer Verbindung der Formel

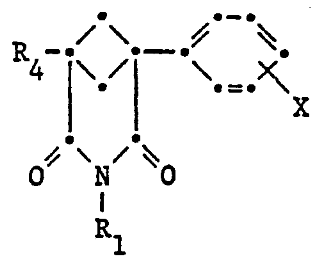

(II),

worin $R_1$ und $R_4$ die unter Formel I genannte Bedeutung haben und X einen Stickstoff enthaltende, reduzierbare Gruppe, Halogen, Carbamoyl, Azidocarbonyl oder eine geschützte Aminogruppe ist, oder in einem Salz davon X in die

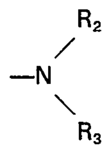

Gruppe überführt, oder

b) eine Verbindung der Formel

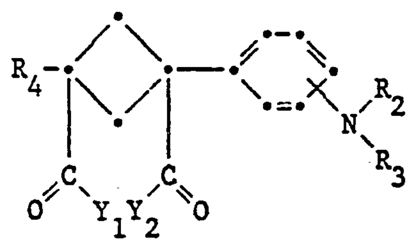

(III),

worin $R_2$ und $R_3$ die unter Formel genannten Bedeutungen haben, $Y_1$ und $Y_2$ unabhängig voneinander Hydroxy, Halogen, Niederalkoxy, Silyloxy oder Sulfonyloxy oder zusammen Sauerstoff darstellen, oder ein Salz davon durch Umsetzung mit einer die Gruppe >N—$R_1$ liefernde Verbindung zyklisiert oder

c) eine Verbindung der Formel

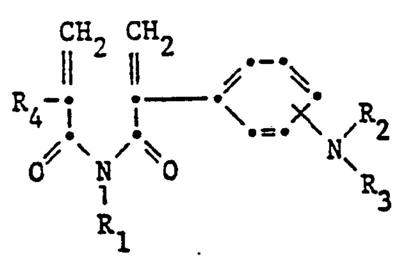

(IV),

worin $R_1$, $R_2$, $R_3$ und $R_4$ die unter Formel I genannte Bedeutung haben, oder ein Salz davon thermisch in Abwesenheit bzw. Anwesenheit eines inerten Lösungsmittels oder auch photochemisch zusammen mit

einem Triplett-Sensibilisatoren in einem photochemisch inerten Lösungsmittel zylkisiert, oder

d) eine Verbindung der Formel

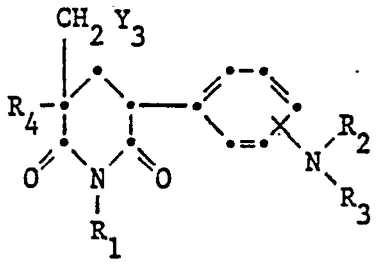

(V)

oder

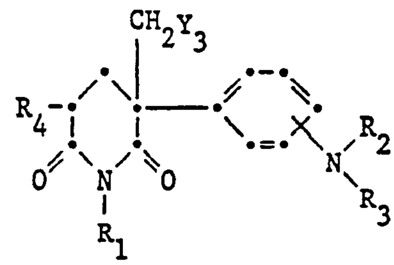

(VI)

worin $R_1$, $R_2$, $R_3$ und $R_4$ die unter Formel I genannte Bedeutung haben und $Y_3$ Halogen oder Sulfonyloxy bedeutet, in Gegenwart einer nicht-nuckleophilen Base zyklisiert und gewünschtenfalls ein erhältliches Salz in die freie Verbindung oder in ein anderes Salz überführt, und/oder eine erhältliche freie Verbindung in ein Salz überführt und/oder ein erhältliches Gemisch von Isomeren in die einzelnen Isomere auftrennt.

2. Verfahren gemäss Anspruch 1, dadurch gekennzeichnet, dass man die Ausgangsstoffe so wählt, dass man eine Verbindung der Formel I, worin $R_1$ Wasserstoff, Alkyl bis einschliesslich 12 C-Atome, Niederalkenyl, Niederalkinyl, Cycloalkyl mit 3—6 C-Atomen, unsubstituiertes Cycloalkylniederalkyl mit 4—7 C-Atomen, oder durch Niederalkyl, Hydroxy, Niederalkoxy, Acyloxy, Amino, Niederalkylamino, Diniederalkylamino, Acylamino oder Halogen substituiertes Arylniederalkyl mit 7—8 C-Atomen, $R_2$ Wasserstoff, Niederalkyl, Niederalkanoyl oder Niederalkansulfonyl, $R_3$ Wasserstoff oder Niederalkyl und $R_4$ Wasserstoff, Niederalkyl, Phenyl oder durch —$N(R_2)(R_3)$ substituiertes Phenyl bedeuten, sowie Salze davon, wobei die mit "nieder" bezeichneten Reste bis einschliesslich 7 Kohlenstoffatome enthalten, herstellt.

3. Verfahren gemäss Anspruch 1, dadurch gekennzeichnet, dass man die Ausgangsstoffe so wählt, dass man eine Verbindung der Formel I, worin $R_1$ Wasserstoff, Niederalkyl, Niederalkenyl, Niederalkinyl, Cycloalkyl mit 3—6 C-Atomen, oder Cycloalkylniederalkyl mit 4—7 C-Atomen, $R_2$ und $R_3$ Wasserstoff und $R_4$ Wasserstoff, Niederalkyl, Phenyl oder durch —$N(R_2)(R_3)$ substituiertes Phenyl bedeuten, sowie pharmazeutisch verwendbare Salze davon, wobei die mit "nieder" bezeichneten Reste bis einschliesslich 7 Kohlenstoffatome enthalten, herstellt.

4. Verfahren gemäss Anspruch 1, dadurch gekennzeichnet, dass man die Ausgangsstoffe so wählt, dass man eine Verbindung der Formel I, worin die Gruppe —$N(R_2)(R_3)$ sich in 4-Stellung des Phenylrestes befindet, $R_1$ Wasserstoff, Niederalkyl, Niederalkenyl, Niederalkinyl oder Cycloalkylniederalkyl mit 4—7 C-Atomen, $R_2$ und $R_3$ Wasserstoff und $R_4$ Wasserstoff oder Niederalkyl bedeuten, sowei pharmazeutisch verwendbare Salze davon, wobei die mit "nieder" bezeichneten Reste bis einschliesslich 7 Kohlenstoffatome enthalten, herstellt.

5. Verfahren gemäss Anspruch 1, dadurch gekennzeichnet, dass man die Ausgangsstoffe so wählt, dass man eine Verbindung der Formel I, worin die Gruppe —$N(R_2)(R_3)$ sich in 4-Stellung des Phenylrests befindet, $R_1$ Wasserstoff, Alkyl bis einschliesslich 12 C-Atome, Niederalkenyl, Niederalkinyl, Cycloalkyl mit 3—6 C-Atomen, Cycloalkylmethyl mit 4—7 C-Atomen oder unsubstituiertes oder substituiertes Benzyl und $R_2$, $R_3$ und $R_4$ Wasserstoff bedeuten, sowie pharmazeutisch verwendbare Salze davon, wobei die mit "nieder" bezeichneten Reste bis einschliesslich 7 Kohlenstoffatome enthalten, herstellt.

6. Verfahren gemäss Anspruch 1, dadurch gekennzeichnet, dass man die Ausgangsstoffe so wählt, dass man 1-(4-Aminophenyl)-3-n-propyl-3-azabicyclo[3.1.1]heptan-2,4-dion herstellt.

7. Verfahren gemäss Anspruch 1, dadurch gekennzeichnet, dass man die Ausgangsstoffe so wählt, dass man 1-(4-Aminophenyl)-3-methyl-3-azabicyclo[3.1.1]heptan-2,4-dion herstellt.

8. Verfahren gemäss Anspruch 1, dadurch gekennzeichnet, dass man die Ausgangsstoffe so wählt, dass man 1-(4-Aminophenyl)-3-n-decyl-3-azabicyclo[3.1.1]heptan-2,4-dion herstellt.

9. Verfahren gemäss Anspruch 1, dadurch gekennzeichnet, dass man die Ausgangsstoffe so wählt, dass man 1-(4-Aminophenyl)-3-cyclohexyl-3-azabicyclo[3.1.1]heptan-2,4-dion herstellt.

10. Verfahren gemäss Anspruch 1, dadurch gekennzeichnet, dass man die Ausgangsstoffe so wählt, dass man 1-(4-Aminophenyl)-3-cyclohexylmethyl-3-azabicyclo[3.1.1]heptan-2,4-dion herstellt.

11. Verfahren zur Herstellung von pharmazeutischen Präparaten, dadurch gekennzeichnet, dass man

Verbindungen der Formel I, worin die Substituenten die im Anspruch 1 genannten Bedeutungen haben, oder Salze davon mit einem Trägerstoff mischt.

12. Verfahren zur Herstellung von Verbindungen der Formel

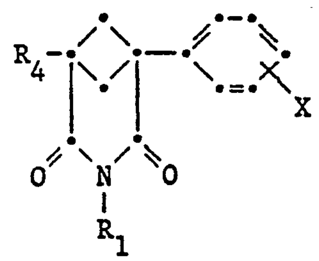

(II),

worin $R_1$ und $R_4$ die unter Formel I in Anspruch 1 genannte Bedeutung haben und X eine im Anspruch 1 unter Formel II definierte Bedeutung hat und Salze von diesen Verbindungen, dadurch gekennzeichnet, dass man

e) eine Verbindung der Formel

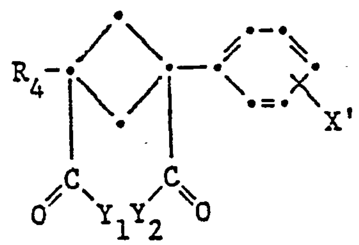

(VII),

worin $R_4$ die unter Formel I im Anspruch 1 genannte Bedeutung hat und $Y_1$ und $Y_2$ unabhängig voneinander Hydroxy, Halogen, Niederalkoxy, Silyloxy oder Sulfonyloxy oder zusammen Sauerstoff und X' eine der unter Formel II in Anspruch 14 angegebenen Gruppen X oder Carboxyl darstellen, oder ein Salz davon durch Umsetzung mit einer die Gruppe

$$\diagdown NHR_1$$

liefernden Verbindung zyklisiert, oder

f) eine Verbindung der Formel

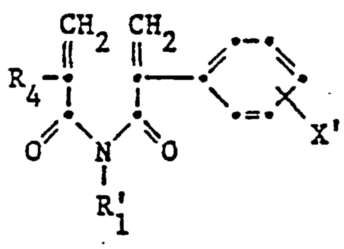

(VIII),

worin $R_1'$ eine Gruppe $R_1$ oder eine Aminoschutzgruppe und X' eine Gruppe X oder Carboxyl darstellen und $R_1$, $R_4$ und X die oben genannten Bedeutungen haben, oder ein Salz davon thermisch in Abwesenheit bzw. Answesenheit eines inerten Lösungsmittel oder auch photochemisch zusammen mit einem Triplett-Sensibilisatoren in einem photochemisch inerten Lösungsmittel zyklisiert

g) eine Verbindung der Formel

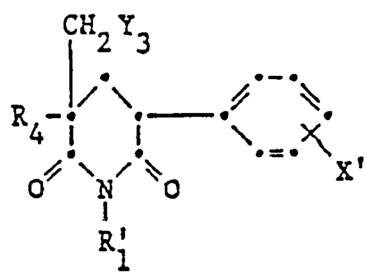

oder

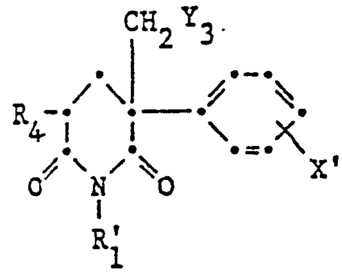

(IX) (X)

worin $R_1'$ eine Gruppe $R_1$ oder eine Aminoschutzgruppe, X' eine Gruppe X oder Carboxyl und $Y_3$ Halogen oder Sulfonyloxy bedeutet, in Gegenwart einer Nicht-nukleophilen Base zyklisiert und in einer erhältlichen Verbindung die Carboxylgruppe X' in Carbamoyl oder Azidocarbonyl überführt und/oder die Aminoschutzgruppe $R_1'$ abspaltet und gewünschtenfalls ein erhältliches Salz in die freie Verbindung oder in ein anderes Salz überführt und/oder eine erhältliche freie Verbindung in ein Salz überführt und/oder ein erhältliches Gemisch von Isomeren in die einzelnen Isomere auftrennt.

13. Verfahren gemäss Anspruch 12, dadurch gekennzeichnet, dass man die Ausgangsstoffe so wählt, dass man eine Verbindung der Formel II, worin $R_1$ und $R_4$ die unter Formel I in Anspruch 1 genannte Bedeutung haben und X eine Nitrogruppe bedeutet, herstellt.

**Claims for the Contracting States: BE CH DE FR GB IT LI LU NL SE**

1. Compounds of the formula

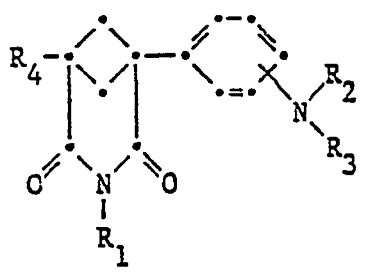

(I),

in which $R_1$ represents hydrogen, alkyl having from 1 to 12 carbon atoms, alkenyl having from 2 to 12 carbon atoms, lower alkynyl, cycloalkyl or cycloalkenyl having from 3 to 10 carbon atoms, cycloalkyl-lower alkyl having from 4 to 10 carbon atoms, cycloalkyl-lower alkenyl having from 5 to 10 carbon atoms, cycloalkenyl-lower alkyl having from 4 to 10 carbon atoms, or unsubstituted or substituted aryl having from 6 to 12 carbon atoms or aryl-lower alkyl having from 7 to 15 carbon atoms, substitution being by lower alkyl, hydroxy, lower alkoxy, acyloxy, amino, lower alkyl-amino, di-lower alkylamino, acylamino or by halogen, $R_2$ represents hydrogen, lower alkyl, sulfo, lower alkanoyl or lower alkanesulfonyl, $R_3$ represents hydrogen or lower alkyl and $R_4$ represents hydrogen, lower alkyl, phenyl or phenyl substituted by $-N(R_2)(R_3)$, and salts thereof, the radicals designated "lower" containing up to and including 7 carbon atoms.

2. Compounds according to claim 1 of the formula I, in which $R_1$ represents hydrogen, alkyl having up to and including 12 carbon atoms, lower alkenyl, lower alkynyl, cycloalkyl having from 3 to 6 carbon atoms, unsubstituted cycloalkyl-lower alkyl having from 4 to 7 carbon atoms, or aryl-lower alkyl having 7 or 8 carbon atoms that is substituted by lower alkyl, hydroxy, lower alkoxy, acyloxy, amino, lower alkylamino, di-lower alkylamino, acylamino or by halogen, $R_2$ represents hydrogen, lower alkyl, lower alkanoyl or lower alkanesulfonyl, $R_3$ represents hydrogen or lower alkyl and $R_4$ represents hydrogen, lower alkyl, phenyl or phenyl substituted by $-N(R_2)(R_3)$, and salts thereof, the radicals designated "lower" containing up to and including 7 carbon atoms.

3. Compounds according to claim 1 of the formula I, in which $R_1$ represents hydrogen, lower alkyl, lower alkenyl, lower alkynyl, cycloalkyl having from 3 to 6 carbon atoms, or cycloalkyl-lower alkyl having from 4 to 7 carbon atoms, $R_2$ and $R_3$ represent hydrogen and $R_4$ represents hydrogen, lower alkyl, phenyl or

phenyl substituted by $-N(R_2)(R_3)$, and pharmaceutically acceptable salts thereof, the radicals designated "lower" containing up to and including 7 carbon atoms.

4. Compounds according to claim 1 of the formula I, in which the group $-N(R_2)(R_3)$ is in the 4-position of the phenyl radical, $R_1$ represents hydrogen, lower alkyl, lower alkenyl, lower alkynyl or cycloalkyl-lower alkyl having from 4 to 7 carbon atoms, $R_2$ and $R_3$ represent hydrogen and $R_4$ represents hydrogen or lower alkyl, and pharmaceutically acceptable salts thereof, the radicals designated "lower" containing up to and including 7 carbon atoms.

5. Compounds according to claim 1 of the formula I, in which the group $-N(R_2)(R_3)$ is in the 4-position of the phenyl radical, $R_1$ represents hydrogen, alkyl having up to and including 12 carbon atoms, lower alkenyl, lower alkynyl, cycloalkyl having from 3 to 6 carbon atoms, cycloalkylmethyl having from 4 to 7 carbon atoms, or unsubstituted or substituted benzyl and $R_2$, $R_3$ and $R_4$ represent hydrogen, and pharmaceutically acceptable salts thereof, the radicals designated "lower" containing up to and including 7 carbon atoms.

6. 1-(4-aminophenyl)-3-n-propyl-3-azabicyclo[3.1.1]heptane-2,4-dione according to claim 1.

7. 1-(4-aminophenyl)-3-methyl-3-azabicyclo[3.1.1]heptane-2,4-dione according to claim 1.

8. 1-(4-aminophenyl)-3-n-decyl-3-azabicyclo[3.1.1]heptane-2,4-dione according to claim 1.

9. 1-(4-aminophenyl)-3-cyclohexyl-3-azabicyclo[3.1.1]heptane-2,4-dione according to claim 1.

10. 1-(4-aminophenyl)-3-cyclohexylmethyl-3-azabicyclo[3.1.1]heptane-2,4-dione according to claim 1.

11. Pharmaceutical preparations containing compounds of the formula I according to claim 1, or salts thereof, together with a carrier.

12. Compounds of the formula I according to claim 1, or salts thereof, for use in a method for the therapeutic treatment of the human or animal body.

13. Compounds of the formula I according to claim 1, or salts thereof, for use as carcinostatic medicaments.

14. Process for the manufacture of compounds of the formula I and salts thereof according to claim 1, characterised in that

a) in a compound of the formula

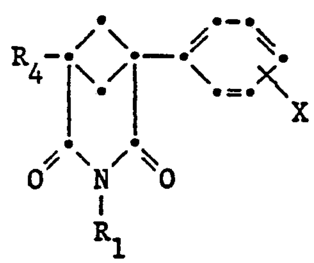

(II),

in which $R_1$ and $R_4$ have the meanings given under formula I and X is a nitrogen-containing reducible group, halogen, carbamoyl, azidocarbonyl or a protected amino group, or in a salt thereof, X is converted into the group

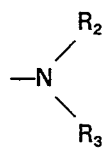

, or

b) a compound of the formula

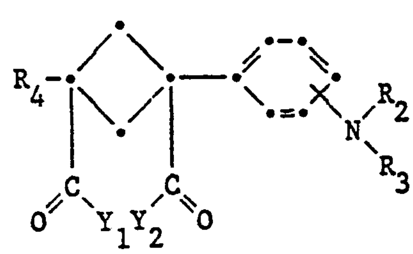

(III),

in which $R_2$, $R_3$ and $R_4$ have the meanings given under formula I and $Y_1$ and $Y_2$, independently of one another, represent hydroxy, halogen, lower alkoxy, silyloxy or sulfonyloxy or together represent oxygen, or

a salt thereof, is cyclised by reaction with a compound that yields the group >N—$R_1$, or

c) a compound of the formula

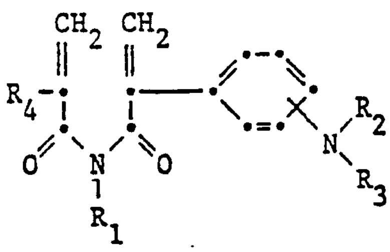

(IV),

in which $R_1$, $R_2$, $R_3$ and $R_4$ have the meanings given under formula I, or a salt thereof, is cyclised thermally in the absence or presence of an inert solvent or photochemically together with a triplet sensitiser in a photochemically inert solvent, or

d) a compound of the formula

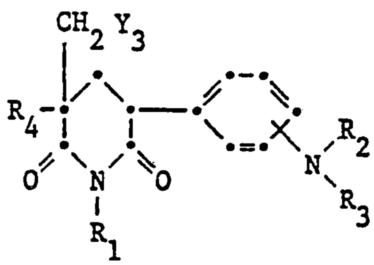

or

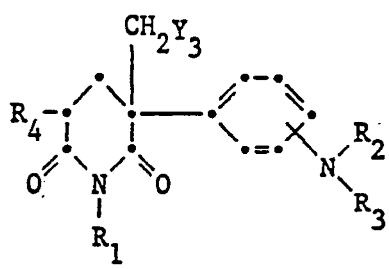

(V) (VI)

in which $R_1$, $R_2$, $R_3$ and $R_4$ have the meanings given under formula I and $Y_3$ represents halogen or sulfonyloxy, is cyclised in the presence of a non-nucleophilic base and, if desired, an obtainable salt is converted into the free compound or into a different salt and/or an obtainable free compound is converted into a salt and/or an obtainable mixture of isomers is separated into the individual isomers.

15. Compounds of the formula

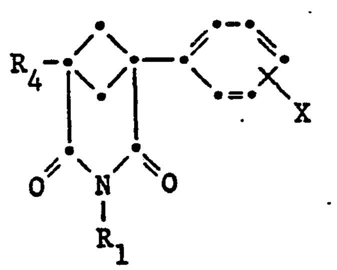

(II),

in which $R_1$ and $R_4$ have the meanings given under formula I in claim 1 and X represents a group defined in claim 14, and salts of these compounds.

16. Compounds according to claim 15 of the formula II in which $R_1$ and $R_4$ have the meanings given under formula I in claim 1 and X represents a nitro group.

17. Process for the manufacture of compounds of the formula II and salts thereof according to claim 15, characterised in that

e) a compound of the formula

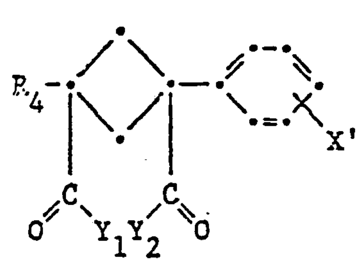

(VII),

in which $R_4$ has the meanings given under formula I in claim 1 and $Y_1$ and $Y_2$, independently of one another, represent hydroxy, halogen, lower alkoxy, silyloxy or sulfonyloxy or together represent oxygen, and X' represents one of the groups X indicated under formula II in claim 14 or carboxy, or a salt thereof, is cyclised by reaction with a compound that yields the group

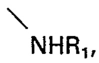, or

f) a compound of the formula

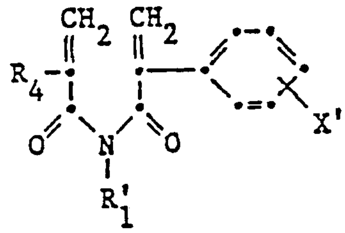

(VIII),

in which $R_1'$ represents a group $R_1$ or an amino-protecting group and X' represents a group X or carboxy, and $R_1$, $R_4$ and X have the meanings given above, or a salt thereof, is cyclised thermally in the absence or presence of an inert solvent or photochemically together with a triplet sensitiser in a photochemically inert solvent, or

g) a compound of the formula

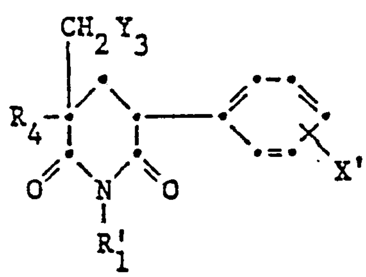

or

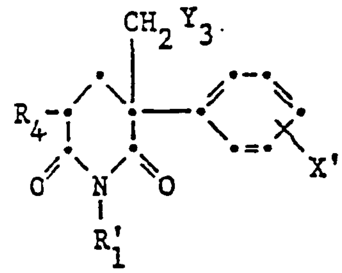

(IX) (X)

in which $R_1'$ represents a group $R_1$ or an amino-protecting group, X' represents a group X or carboxy and $Y_3$ represents halogen or sulfonyloxy, is cyclised in the presence of a non-nucleophilic base and in an obtainable compound the carboxy group X' is converted into carbamoyl or azidocarbonyl and/or the amino-protecting group $R_1'$ is removed and, if desired, an obtainable salt is converted into the free compound or into a different salt and/or an obtainable free compound is converted into a salt and/or an obtainable mixture of isomers is separated into the individual isomers.

18. Use of compounds of the formula I according to claim 1 for the manufacture of a medicament for the treatment of hormone-dependent tumours and hormonal anomalies.

**Claims for the Contracting State: AT**

1. Process for the manufacture of compounds of the formula

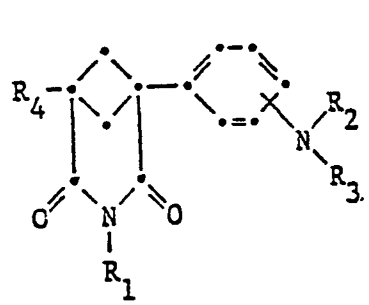

(I),

in which $R_1$ represents hydrogen, alkyl having from 1 to 12 carbon atoms, alkenyl having from 2 to 12 carbon atoms, lower alkynyl, cycloalkyl or cycloalkenyl having from 3 to 10 carbon atoms, cycloalkyl-lower alkyl having from 4 to 10 carbon atoms, cycloalkyl-lower alkenyl having from 5 to 10 carbon atoms,

cycloalkenyl-lower alkyl having from 4 to 10 carbon atoms, or unsubstituted or substituted aryl having from 6 to 12 carbon atoms or aryl-lower alkyl having from 7 to 15 carbon atoms, substitution being by lower alkyl, hydroxy, lower alkoxy, acyloxy, amino, lower alkylamino, di-lower alkylamino, acylamino or by halogen, $R_2$ represents hydrogen, lower alkyl, sulfo, lower alkanoyl or lower alkanesulfonyl, $R_3$ represents hydrogen or lower alkyl and $R_4$ represents hydrogen, lower alkyl, phenyl or phenyl substituted by $—N(R_2)(R_3)$, and salts thereof, the radicals designated "lower" containing up to and including 7 carbon atoms, characterised in that

a) in a compound of the formula

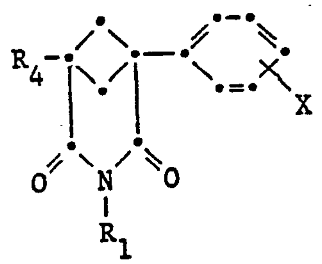

(II),

in which $R_1$ and $R_4$ have the meanings given under formula I and X is a nitrogen-containing reducible group, halogen, carbamoyl, azidocarbonyl or a protected amino group, or in a salt thereof, X is converted into the group

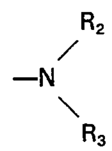

, or

b) a compound of the formula

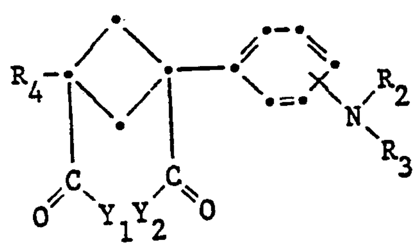

(III),

in which $R_2$ and $R_3$ have the meanings given under formula I and $Y_1$ and $Y_2$, independently of one another, represent hydroxy, halogen, lower alkoxy, silyloxy or sulfonyloxy or together represent oxygen, or a salt thereof, is cyclised by reaction with a compound that yields the group $>N—R_1$, or

c) a compound of the formula

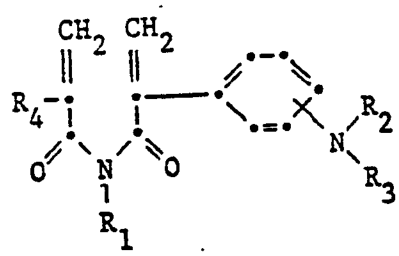

(IV),

in which $R_1$, $R_2$, $R_3$ and $R_4$ have the meanings given under formula I, or a salt thereof, is cyclised thermally in the absence or presence of an inert solvent or photochemically together with a triplet sensitiser in a photochemically inert solvent, or

d) a compound of the formula

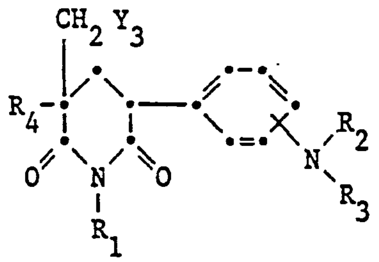

(V)

or

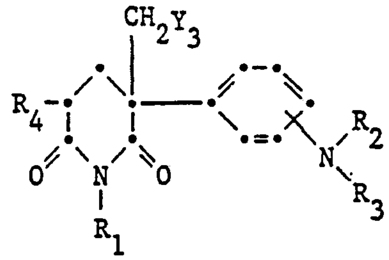

(VI)

in which $R_1$, $R_2$, $R_3$ and $R_4$ have the meanings given under formula I and $Y_3$ represents halogen or sulfonyloxy, is cyclised in the presence of a non-nucleophilic base and, if desired, an obtainable salt is converted into the free compound or into a different salt and/or an obtainable free compound is converted into a salt and/or an obtainable mixture of isomers is separated into the individual isomers.

2. Process according to claim 1, characterised in that the starting materials are so chosen that a compound of the formula I, in which $R_1$ represents hydrogen, alkyl having up to and including 12 carbon atoms, lower alkenyl, lower alkynyl, cycloalkyl having from 3 to 6 carbon atoms, unsubstituted cycloalkyl-lower alkyl having from 4 to 7 carbon atoms, or aryl-lower alkyl having 7 or 8 carbon atoms that is substituted by lower alkyl, hydroxy, lower alkoxy, acyloxy, amino, lower alkylamino, di-lower alkylamino, acylamino or by halogen, $R_2$ represents hydrogen, lower alkyl, lower alkanoyl or lower alkanesulfonyl, $R_3$ represents hydrogen or lower alkyl and $R_4$ represents hydrogen, lower alkyl, phenyl or phenyl substituted by $-N(R_2)(R_3)$, and salts thereof, the radicals designated "lower" containing up to and including 7 carbon atoms, are manufactured.

3. Process according to claim 1, characterised in that the starting materials are so chosen that a compound of the formula I in which $R_1$ represents hydrogen, lower alkyl, lower alkenyl, lower alkynyl, cycloalkyl having from 3 to 6 carbon atoms, or cycloalkyl-lower alkyl having from 4 to 7 carbon atoms, $R_2$ and $R_3$ represent hydrogen and $R_4$ represents hydrogen, lower alkyl, phenyl or phenyl substituted by $-N(R_2)(R_3)$, and pharmaceutically acceptable salts thereof, the radicals designated "lower" containing up to and including 7 carbon atoms, are manufactured.

4. Process according to claim 1, characterised in that the starting materials are so chosen that a compound of the formula I in which the group $-N(R_2)(R_3)$ is in the 4-position of the phenyl radical, $R_1$ represents hydrogen, lower alkyl, lower alkenyl, lower alkynyl or cycloalkyl-lower alkyl having from 4 to 7 carbon atoms, $R_2$ and $R_3$ represent hydrogen and $R_4$ represents hydrogen or lower alkyl, and pharmaceutically acceptable salts thereof, the radicals designated "lower" containing up to and including 7 carbon atoms, are manufactured.

5. Process according to claim 1, characterised in that the starting materials are so chosen that a compound of the formula I in which the group $-N(R_2)(R_3)$ is in the 4-position of the phenyl radical, $R_1$ represents hydrogen, alkyl having up to and including 12 carbon atoms, lower alkenyl, lower alkynyl, cycloalkyl having from 3 to 6 carbon atoms, cycloalkylmethyl having from 4 to 7 carbon atoms, or unsubstituted or substituted benzyl and $R_2$, $R_3$ and $R_4$ represent hydrogen, and pharmaceutically acceptable salts thereof, the radicals designated "lower" containing up to and including 7 carbon atoms, are manufactured.

6. Process according to claim 1, characterised in that the starting materials are so chosen that 1-(4-aminophenyl)-3-n-propyl-3-azabicyclo[3.1.1]heptane-2,4-dione is manufactured.

7. Process according to claim 1, characterised in that the starting materials are so chosen that 1-(4-aminophenyl)-3-methyl-3-azabicyclo[3.1.1]heptane-2,4-dione is manufactured.

8. Process according to claim 1, characterised in that the starting materials are so chosen that 1-(4-aminophenyl)-3-n-decyl-3-azabicyclo[3.1.1]heptane-2,4-dione is manufactured.

9. Process according to claim 1, characterised in that the starting materials are so chosen that 1-(4-aminophenyl)-3-cyclohexyl-3-azabicyclo[3.1.1]heptane-2,4-dione is manufactured.

10. Process according to claim 1, characterised in that the starting materials are so chosen that 1-(4-aminophenyl)-3-cyclohexylmethyl-3-azabicyclo[3.1.1]heptane-2,4-dione is manufactured.

11. Process for the manufacture of pharmaceutical preparations, characterised in that compounds of

the formula I in which the substituents have the meanings given in claim 1, or salts thereof, are mixed with a carrier.

12. Process for the manufacture of compounds of the formula

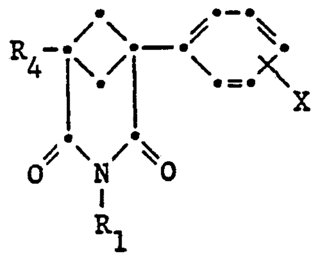

(II),

in which $R_1$ and $R_4$ have the meanings given under formula I in claim 1 and X has one of the meanings defined under formula II in claim 1, and salts of these compounds, characterised in that

e) a compound of the formula

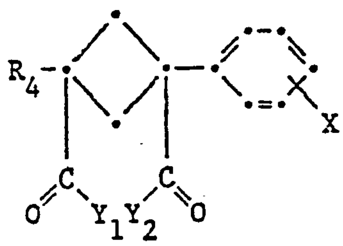

(VII),

in which $R_4$ has the meanings given under formula I in claim 1 and $Y_1$ and $Y_2$, independently of one another, represent hydroxy, halogen, lower alkoxy, silyloxy or sulfonyloxy or together represent oxygen, and X' represents one of the groups X indicated under formula II in claim 1 or carboxy, or a salt thereof, is cyclised by reaction with a compound that yields the group

NH$R_1$, or

f) a compound of the formula

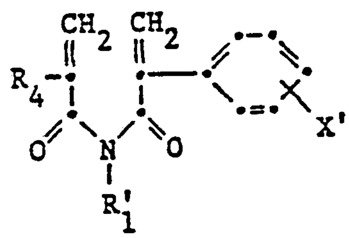

(VIII),

in which $R_1'$ represents a group $R_1$ or an amino-protecting group and X' represents a group X or carboxy, and $R_1$, $R_4$ and X have the meanings given above, or a salt thereof, is cyclised thermally in the absence or presence of an inert solvent or photochemically together with a triplet sensitiser in a photochemically inert solvent, or

g) a compound of the formula

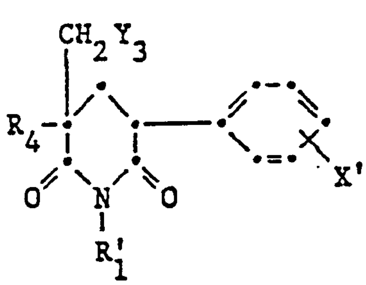

or

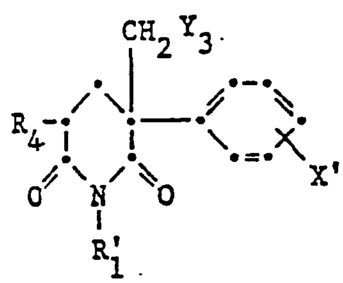

(IX) (X)

in which $R_1'$ represents a group $R_1$ or an amino-protecting group, X' represents a group X or carboxy and $Y_3$ represents halogen or sulfonyloxy, is cyclised in the presence of a non-nucleophilic base and in an obtainable compound the carboxy group X' is converted into carbamoyl or azidocarbonyl and/or the amino-protecting group $R_1'$ is removed and, if desired, an obtainable salt is converted into the free compound or into a different salt and/or an obtainable free compound is converted into a salt and/or an obtainable mixture of isomers is separated into the individual isomers.

13. Process according to claim 12, characterised in that the starting materials are so chosen that a compound of the formula II in which $R_1$ and $R_4$ have the meanings given under formula I in claim 1 and X represents a nitro group, is manufactured.

**Revendications pour les Etats contractants: BE CH DE FR GB IT LI LU NL SE**

1. Composés de formule

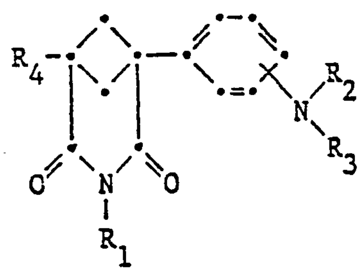

(I),

dans laquelle $R_1$ représente l'hydrogène, un groupe alkyle en C1—C12, alcényle en C2—C12, alcynyle inférieur, cycloalkyle ou cycloalcényle en C3—C10, cycloalkyl-alkyle inférieur en C4—C10, cycloalkyl-alcényle inférieur en C5—C10, cycloalcényl-alkyle inférieur en C4—C10 ou aryle en C6—C12 ou aryle-alkyle inférieur en C7—C15 non substitué ou substitué par des groupes alkyle inférieurs, hydroxy, alcoxy inférieurs, acyloxy, amino, alkylamino inférieurs, di-(alkyle inférieur)-amino, acylamino ou des halogènes, $R_2$ représente l'hydrogène, un groupe alkyle inférieur, sulfo, alcanoyle inférieur ou alcane-sulfonyle inférieur, $R_3$ représente l'hydrogène ou un groupe alkyle inférieur et $R_4$ représente l'hydrogène, un groupe alkyle inférieur, phényle ou phényle substitué par —$N(R_2)(R_3)$, et leurs sels, l'expression "inférieurs" s'appliquant à des groupes qui contiennent jusqu'à 7 atomes de carbone inclus.

2. Composés de la revendication 1, de formule I dans laquelle $R_1$ représente l'hydrogène, un groupe alkyle contenant jusqu'à 12 atomes de carbone inclus, un groupe alcényle inférieur, alcynyle inférieur, cycloalkyle en C3—C6, cycloalkyl-alkyle inférieur non substitué en C4—C7 ou aryl-alkyle inférieur en C7—C8 substitué par des groupes alkyle inférieurs, hydroxy, alcoxy inférieurs, acyloxy, amino, alkylamino inférieurs, di-(alkyle inférieur)-amino, acylamino ou des halogènes, $R_2$ représente l'hydrogène, un groupe alkyle inférieur, alcanoyle inférieur ou alcane-sulfonyle inférieur, $R_3$ représente l'hydrogène ou un groupe alkyle inférieur et $R_4$ représente l'hydrogène, un groupe alkyle inférieur, phényle ou phényle substitué par —$N(R_2)(R_3)$, et leurs sels, l'expression "inférieurs" s'appliquant à des groupes qui contiennent jusqu'à 7 atomes de carbone inclus.

3. Composés selon la revendication 1, de formule I dans laquelle $R_1$ représente l'hydrogène, un groupe alkyle inférieur, alcényle inférieur, alcynyle inférieur, cycloalkyle en C3—C6 ou cycloalkyl-alkyle inférieur en C4—C7, $R_2$ et $R_3$ représentent l'hydrogène et $R_4$ représente l'hydrogène, un groupe alkyle inférieur, phényle ou phényle substitué par —$N(R_2)(R_3)$, et leurs sels acceptables pour l'usage pharmaceutique, l'expression "inférieurs" s'appliquant à des groupes qui contiennent jusqu'à 7 atomes de carbone inclus.

4. Composés selon la revendication 1, de formule I dans laquelle le groupe —$N(R_2)(R_3)$ se trouve en position 4 du groupe phényle, $R_1$ représente l'hydrogène, un groupe alkyle inférieur, alcényle inférieur, alcynyle inférieur ou cycloalkyl-alkyle inférieur en C4—C7, $R_2$ et $R_3$ représentent l'hydrogène et $R_4$ représente l'hydrogène ou un groupe alkyle inférieur, et leurs sels acceptables pour l'usage pharmaceutique, l'expression "inférieurs" s'appliquant à des groupes qui contiennent jusqu'à 7 atomes de carbone inclus.

5. Composés selon la revendication 1, de formule I dans laquelle le groupe —$N(R_2)(R_3)$ se trouve en position 4 du groupe phényle, $R_1$ représente l'hydrogène, un groupe alkyle contenant jusqu'à 12 atomes de carbone inclus, un groupe alcényle inférieur, alcynyle inférieur, cycloalkyle en C3—C6, cycloalkyl-méthyle en C4—C7, ou benzyle non substitué ou substitué et $R_2$, $R_3$ et $R_4$ représentant l'hydrogène, et leurs sels acceptables pour l'usage pharmaceutique, l'expression "inférieurs" s'appliquant à des groupes qui contiennent jusqu'à 7 atomes de carbone inclus.

6. La 1-(4-aminophényl)-3-n-propyl-3-azabicyclo[3.1.1]heptane-2,4-dione selon la revendication 1.

7. La 1-(4-aminophényl)-3-méthyl-3-azabicyclo[3.1.1]heptane-2,4-dione selon la revendication 1.

8. La 1-(4-aminophényl)-3-n-décyl-3-azabicyclo[3.1.1]heptane-2,4-dione selon la revendication 1.

9. La 1-(4-aminophényl)-3-cyclohexyl-3-azabicyclo[3.1.1]heptane-2,4-dione selon la revendication 1.

10. La 1-(4-aminophényl)-3-cyclohexylméthyl-3-azabicyclo[3.1.1]heptane-2,4-dione selon la revendication 1.

11. Compositions pharmaceutiques contenant des composés de formule I selon la revendication 1 ou des sels de tels composés avec un véhicule.

12. Composés de formule I selon la revendication 1 ou leurs sels, pour l'utilisation dans un procédé pour le traitement thérapeutique de l'organisme humain ou animal.

13. Composés de formule I selon la revendication 1 ou leurs sels, pour l'utilisation en tant que médicaments carcinostatiques.

14. Procédé de préparation des composés de formule I et de leurs sels selon la revendication 1, caractérisé en ce que:

a) dans un composé de formule

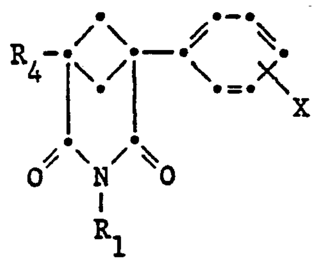

(II),

dans laquelle $R_1$ et $R_4$ ont les significations indiquées en référence à la formule I et X représente un groupe réductible contenant de l'azote, un halogène, un groupe carbamoyle, azidocarbonyle ou un groupe amino protégé, ou dans un sel d'un tel composé, on convertit X en le groupe

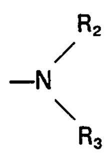

, ou bien

b) on cyclise un composé de formule

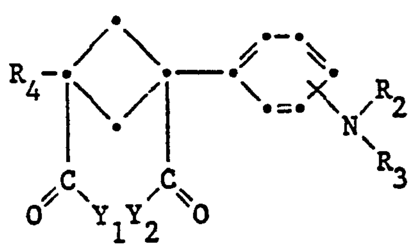

(III),

dans laquelle $R_2$, $R_3$ et $R_4$ ont les significations indiquées en référence à la formule I, $Y_1$ et $Y_2$ représentent chacun, indépendamment l'un de l'autre, un groupe hydroxy, un halogène, un groupe alcoxy inférieur, silyloxy ou sulfonyloxy ou représentent ensemble l'oxygène, ou un sel d'un tel composé, par réaction avec un composé apportant le groupe $>N—R_1$, ou bien

c) on cyclise un composé de formule

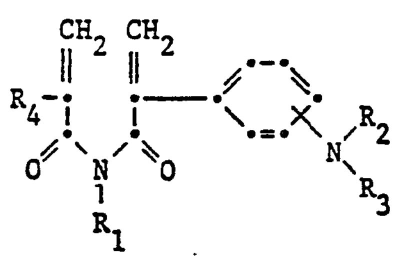

(IV),

dans laquelle $R_1$, $R_2$, $R_3$ et $R_4$ ont les significations indiquées en référence à la formule I, ou un sel d'un tel composé, à la chaleur, en l'absence ou en présence d'un solvant inerte, ou par voie photochimique, avec un

sensibilisant à triplet dans un solvant inerte du point de vue photochimique, ou bien

d) on cyclise un composé de formule

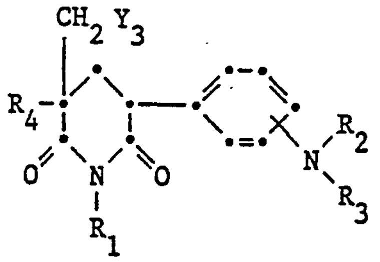

(V)

ou

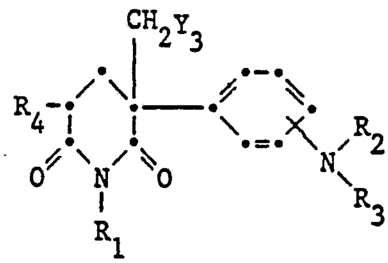

(VI)

dans laquelle $R_1$, $R_2$, $R_3$ et $R_4$ ont les significations indiquées en référence à la formule I et $Y_3$ représente un halogène ou un groupe sulfonyloxy, en présence d'une base non nucléophile, et si on le désire, on convertit un sel ainsi obtenu en le composé libre ou en un autre sel, et/ou un composé obtenu à l'état libre en un sel et/ou on résout un mélange d'isomères ainsi obtenu en les isomères individuels.

15. Composés de formule

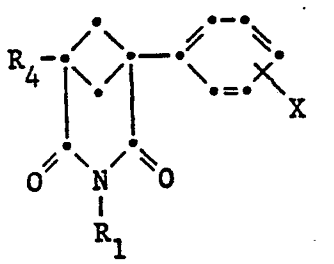

(II),

dans laquelle $R_1$ et $R_4$ ont les significations indiquées en référence à la formule I dans la revendication 1 et X représente un groupe défini dans la revendication 14, et les sels de ces composés.

16. Composés selon la revendication 15, de formule II dans laquelle $R_1$ et $R_4$ ont les significations indiquées en référence à la formule I dans la revendication 1 et X représente un groupe nitro.

17. Procédé de préparation des composés de formule II et de leurs sels selon la revendication 15, caractérisé en ce que:

e) on cyclise un composé de formule

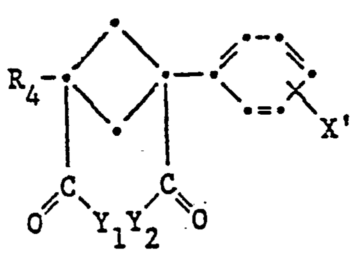

(VII),

dans laquelle $R_4$ a les significations indiquées en référence à la formule I dans la revendication 1 et $Y_1$ et $Y_2$ représentent chacun, indépendamment l'un de l'autre, un groupe hydroxy, un halogène, un groupe alcoxy inférieur, silyloxy ou sulfonyloxy ou représentent ensemble l'oxygène et X' représente l'un des groupes X définis en référence à la formule II dans la revendication 14 ou un groupe carboxyle, ou un sel d'un tel composé, par réaction avec un composé apportant le groupe

$NHR_1$, ou bien

f) on cyclise un composé de formule

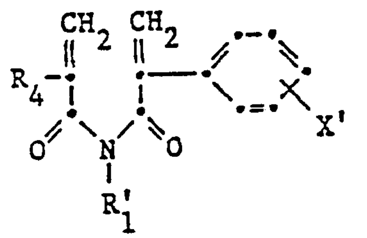

(VIII),

dans laquelle $R'_1$ représente un groupe $R_1$ ou un groupe protecteur du groupe amino et X′ représente un groupe X ou un groupe carboxyle, et $R_1$, $R_4$ et X ont les significations indiquées ci-dessus, ou un sel d'un tel composé, à la chaleur, en l'absence ou en présence d'un solvant inerte, ou par voie photochimique avec un sensibilisant à triplet dans un solvant inerte du point de vue photochimique,

g) on cyclise un composé de formule

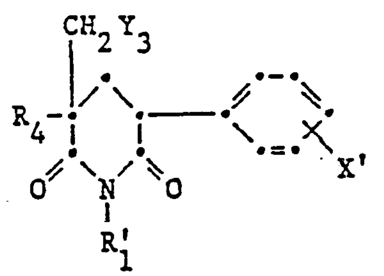

ou

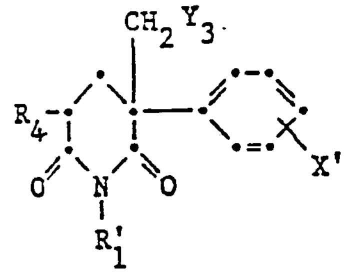

(IX) (X)

dans laquelle $R'_1$ représente un groupe $R_1$ ou un groupe protecteur du groupe amino, X′ représente un groupe X ou un groupe carboxyle et $Y_3$ représente un halogène ou un groupe sulfonyloxy, en présence d'une base non nucléophile, et dans un composé ainsi obtenu, on convertit le groupe carboxyle X′ en groupe carbamoyle ou azidocarbonyle et/ou on élimine le groupe protecteur du groupe amino $R'_1$ et si on le désire, on convertit un sel ainsi obtenu en le composé libre ou en un autre sel et/ou un composé ainsi obtenu à l'état libre en un sel et/ou on résout un mélange d'isomères ainsi obtenu en les isomères individuels.

18. Utilisation des composés de formule I selon la revendication 1, pour la préparation d'un médicament pour le traitement des tumeurs en relation avec les hormones et des anomalies hormonales.

**Revendications pour l'Etat contractant: AT**

1. Procédé de préparation des composés de formule

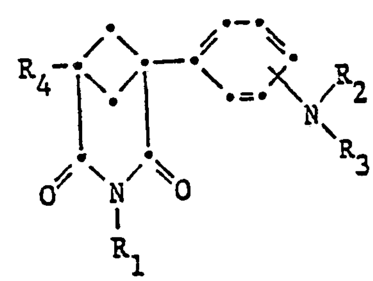

(I),

dans laquelle $R_1$ représente l'hydrogène, un groupe alkyle en C1—C12, alcényle en C2—C12, alcynyle inférieur, cycloalkyle ou cycloalcényle en C3—C10, cycloalkyl-alkyle inférieur en C4—C10, cycloalkyl-alcényle inférieur en C5—C10, cycloalcényl-alkyle inférieur en C4—C10, ou un groupe aryle en C6—C12 ou aryl-alkyle inférieur en C7—C15 non substitué ou substitué par des groupes alkyle inférieurs, hydroxy, alcoxy inférieurs, acyloxy, amino, alkylamino inférieurs, di-(alkyle inférieur)-amino, acylamino ou des halogènes, $R_2$ représente l'hydrogène, un groupe alkyle inférieur, sulfo, alcanoyle inférieur ou alcane-sulfonyle inférieur, $R_3$ représente l'hydrogène ou un groupe alkyle inférieur et $R_4$ représente l'hydrogène, un groupe alkyle inférieur, phényle ou phényle substitué par —N($R_2$)($R_3$), et de leurs sels, l'expression

"inférieurs" s'appliquant à des groupes qui contiennent jusqu'à 7 atomes de carbone inclus, caractérisé en ce que:

a) dans un composé de formule

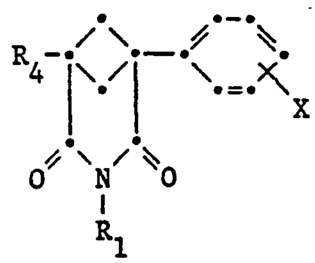

(II),

dans laquelle $R_1$ et $R_4$ ont les significations indiquées en référence à la formule I et X représente un groupe réductible contenant de l'azote, un halogène, un groupe carbamoyle, azidocarbonyle ou un groupe amino protégé, ou dans un sel d'un tel composé, on convertit X en le groupe

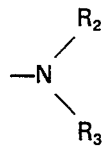

, ou bien

b) on cyclise un composé de formule

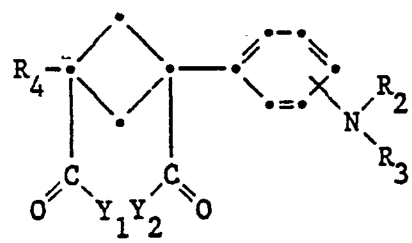

(III),

dans laquelle $R_2$ et $R_3$ ont les significations indiquées en référence à la formule I, $Y_1$ et $Y_2$ représentent chacun, indépendamment l'un de l'autre, un groupe hydroxy, un halogène, un groupe alcoxy inférieur, silyloxy ou sulfonyloxy ou représentent ensemble l'oxygène, ou un sel d'un tel composé, par réaction avec un composé apportant le groupe >N—$R_1$, ou bien

c) on cyclise un composé de formule

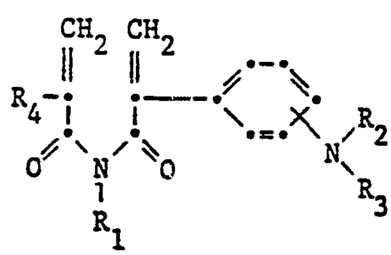

(IV),

dans laquelle $R_1$, $R_2$, $R_3$ et $R_4$ ont les significations indiquées en référence à la formule I, ou un sel d'un tel composé, à la chaleur en l'absence ou en présence d'un solvant inerte, ou par voie photochimique avec un sensibilisant à triplet dans un solvant inerte du point de vue photochimique, ou bien

d) on cyclise un composé de formule

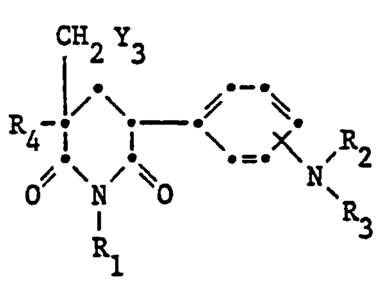

(V)

ou

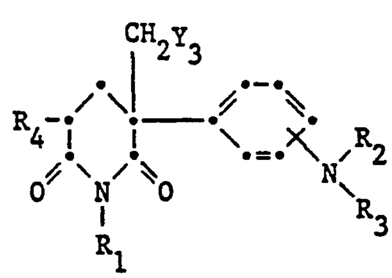

(VI)

dans laquelle $R_1$, $R_2$, $R_3$ et $R_4$ ont les significations indiquées en référence à la formule I et $Y_3$ représente un halogène ou un groupe sulfonyloxy, en présence d'une base non nucléophile, et si on le désire, on convertit un sel ainsi obtenu en le composé libre ou en un autre sel, et/ou on convertit un composé ainsi obtenu à l'état libre en un sel et/ou on sépare un mélange d'isomères ainsi obtenu en les isomères individuels.

2. Procédé selon la revendication 1, caractérisé en ce que l'on choisit les composants de départ de manière à obtenir un composé de formule I dans laquelle $R_1$ représente l'hydrogène, un groupe alkyle contenant jusqu'à 12 atomes de carbone inclus, un groupe alcényle inférieur, alcynyle inférieur, cycloalkyle en C3—C6, cycloalkyl-alkyle inférieur non substitué en C4—C7 ou aryl-alkyle inférieur en C7 ou C8 substitué par des groupes alkyle inférieurs, hydroxy, alcoxy inférieurs, acyloxy, amino, alkylamino inférieurs, di-(alkyle inférieur)-amino, acylamino ou des halogènes, $R_2$ représente l'hydrogène, un groupe alkyle inférieur, alcanoyle inférieur ou alcane-sulfonyle inférieur, $R_3$ représente l'hydrogène ou un groupe alkyle inférieur et $R_4$ représente l'hydrogène, un groupe alkyle inférieur, phényle ou phényle substitué par $—N(R_2)(R_3)$, et leurs sels, l'expression "inférieurs" s'appliquant à des groupes qui contiennent jusqu'à 7 atomes de carbone inclus.

3. Procédé selon la revendication 1, caractérisé en ce que l'on choisit les composants de départ de manière à obtenir un composé de formule I dans laquelle $R_1$ représente l'hydrogène, un groupe alkyle inférieur, alcényle inférieur, alcynyle inférieur, cycloalkyle en C3—C6 ou cycloalkyl-alkyle inférieur en C4—C7, $R_2$ et $R_3$ représentent l'hydrogène et $R_4$ l'hydrogène, un groupe alkyle inférieur, phényle ou phényle substitué par $—N(R_2)(R_3)$, et leurs sels acceptables pour l'usage pharmaceutique, l'expression "inférieurs" s'appliquant à des groupes qui contiennent jusqu'à 7 atomes de carbone inclus.

4. Procédé selon la revendication 1, caractérisé en ce que l'on choisit les composants de départ de manière à obtenir un composé de formule I dans laquelle le groupe $—N(R_2)(R_3)$ se trouve en position 4 du groupe phényle, $R_1$ représente l'hydrogène, un groupe alkyle inférieur, alcényle inférieur, alcynyle inférieur ou cycloalkyl-alkyle inférieur en C4—C7, $R_2$ et $R_3$ représentent l'hydrogène et $R_4$ l'hydrogène ou un groupe alkyle inférieur, et leurs sels acceptables pour l'usage pharmaceutique, l'expression "inférieurs" s'appliquant à des groupes qui contiennent jusqu'à 7 atomes de carbone inclus.

5. Procédé selon la revendication 1, caractérisé en ce que l'on choisit les composants de départ de manière à obtenir un composé de formule I dans laquelle le groupe $—N(R_2)(R_3)$ se trouve en position 4 du groupe phényle, $R_1$ représente l'hydrogène, un groupe alkyle contenant jusqu'à 12 atomes de carbone inclus, alcényle inférieur, alcynyle inférieur, cycloalkyle en C3—C6, cycloalkyl-méthyle en C4—C7 ou benzyle non substitué ou substitué et $R_2$, $R_3$ et $R_4$ représentent l'hydrogène, et leurs sels acceptables pour l'usage pharmaceutique, l'expression "inférieurs" s'appliquant à des groupes qui contiennent jusqu'à 7 atomes de carbone inclus.

6. Procédé selon la revendication 1, caractérisé en ce que l'on choisit les composants de départ de manière à obtenir la 1-(4-aminophényl)-3-n-propyl-3-azabicyclo[3.1.1]heptane-2,4-dione.

7. Procédé selon la revendication 1, caractérisé en ce que l'on choisit les composants de départ de manière à obtenir la 1-(4-aminophényl)-3-méthyl-3-azabicyclo[3.1.1]heptane-2,4-dione.

8. Procédé selon la revendication 1, caractérisé en ce que l'on choisit les composants de départ de manière à obtenir la 1-(4-aminophényl)-3-n-décyl-3-azabicyclo[3.1.1]heptane-2,4-dione.

9. Procédé selon la revendication 1, caractérisé en ce que l'on choisit les composants de départ de manière à obtenir la 1-(4-aminophényl)-3-cyclohexyl-3-azabicyclo[3.1.1]heptane-2,4-dione.

10. Procédé selon la revendication 1, caractérisé en ce que l'on choisit les composants de départ de manière à obtenir la 1-(4-aminophényl)-3-cyclohexylméthyl-3-azabicyclo[3.1.1]heptane-2,4-dione.

11. Procédé de préparation de compositions pharmaceutiques, caractérisé en ce que l'on mélange des composés de formule I dans laquelle les symboles ont les significations indiquées dans la revendication 1, ou des sels de tels composés, avec un véhicule.

12. Procédé de préparation de composés de formule

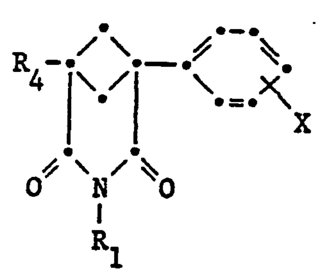

(II),

dans laquelle $R_1$ et $R_4$ ont les significations indiquées en référence à la formule I dans la revendication 1 et X a l'une des significations indiquées dans la revendication 1 en référence à la formule II, et des sels de ces composés, caractérisé en ce que:

e) on cyclise un composé de formule

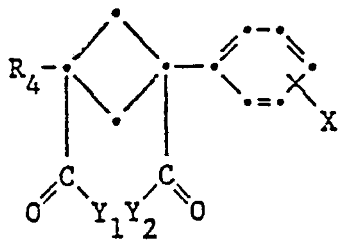

(VII),

dans laquelle $R_4$ a les significations indiquées dans la revendication 1 en référence à la formule I et $Y_1$ et $Y_2$ représentent chacun, indépendamment l'un de l'autre, un groupe hydroxy, un halogène, un groupe alcoxy inférieur, silyloxy ou sulfonyloxy ou représentent ensemble l'oxygène, et X' représente l'un des groupes X définis dans la revendication 1 en référence à la formule II, ou un groupe carboxyle, ou un sel d'un tel composé, par réaction avec un composé apportant le groupe

\ $NHR_1$, ou bien

f) on cyclise un composé de formule

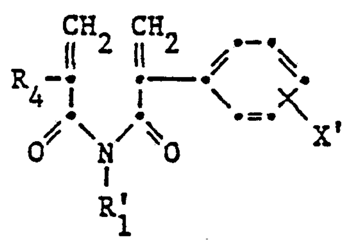

(VIII),

dans laquelle $R_1'$ représente un groupe $R_1$ ou un groupe protecteur du groupe amino et X' représente un groupe X ou un groupe carboxyle, et $R_1$, $R_4$ et X ont les significations indiquées ci-dessus, ou un sel d'un tel composé, à la chaleur, en l'absence ou en présence d'un solvant inerte, ou encore par voie photochimique, avec un sensibilisant à triplet, dans un solvant inerte du point de vue photochimique;

g) on cyclise un composé de formule

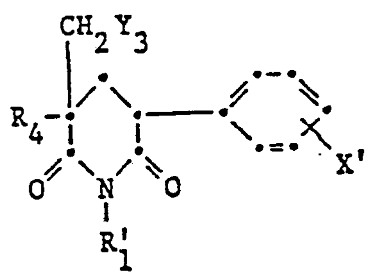

ou

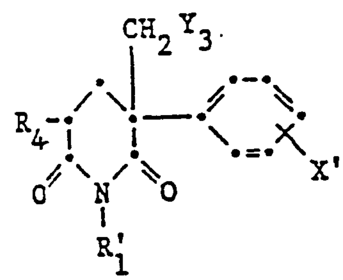

(IX) (X)

dans laquelle $R_1'$ représente un groupe $R_1$ ou un groupe protecteur du groupe amino, X' représente un groupe X ou un groupe carboxyle et $Y_3$ représente un halogène ou un groupe sulfonyloxy, en présence d'une base non nucléophile, et dans un composé ainsi obtenu, on convertit le groupe carboxyle X' en groupe carbamoyle ou azidocarbonyle, et/ou on élimine le groupe protecteur du groupe amino $R_1'$, et si on le désire, on convertit un sel ainsi obtenu en le composé libre ou en un autre sel et/ou un composé ainsi obtenu à l'état libre en un sel et/ou on sépare un mélange d'isomères ainsi obtenu en les isomères individuels.

13. Procédé selon la revendication 12, caractérisé en ce que l'on choisit les composants de départ de manière à obtenir un composé de formule II dans laquelle $R_1$ et $R_4$ ont les significations indiquées dans la revendication 1 en référence à la formule I et X représente un groupe nitro.